(19) Europäisches Patentamt
European Patent Office
Office européen des brevets

(11) Veröffentlichungsnummer: **0 374 097**
**A2**

# (12) EUROPÄISCHE PATENTANMELDUNG

(21) Anmeldenummer: **89810922.8**

(22) Anmeldetag: **06.12.89**

(51) Int. Cl.5: **C07K 5/02, C07K 5/06, C07K 7/02, A61K 37/64**

(30) Priorität: **15.12.88 CH 4641/88**

(43) Veröffentlichungstag der Anmeldung: **20.06.90 Patentblatt 90/25**

(84) Benannte Vertragsstaaten: **AT BE CH DE FR GB IT LI LU NL SE**

(71) Anmelder: **CIBA-GEIGY AG**
**Klybeckstrasse 141**
**CH-4002 Basel(CH)**

(72) Erfinder: **Fässler, Alexander, Dr.**
**Hallenstrasse 10**
**CH-4104 Oberwil(CH)**
Erfinder: **Hungerbühler, Ernst, Dr.**
**Zeisigweg 4**
**CH-4310 Rheinfelden(CH)**
Erfinder: **Rösel, Johannes, Dr.**
**Grenzacherweg 78**
**CH-4125 Riehen(CH)**
Erfinder: **Rüeger, Heinrich, Dr.**
**Alemannenweg 6**
**CH-4112 Flüh(CH)**
Erfinder: **Schneider, Peter, Dr.**
**Bäumliackerstrasse 8**
**CH-4103 Bottmingen(CH)**
Erfinder: **Wood, Jeanette, Dr.**
**Mühleweg 15**
**CH-4105 Biel-Benken(CH)**

## (54) Verwendung von Peptidisosteren als retrovirale Proteasehemmer.

(57) Die Erfindung betrifft die Verwendung von Verbindungen vom Typus der Renininhibitoren oder verwandter Aspartatproteinaseinhibitoren als gag-Protease-Inhibitoren. Sie betrifft insbesondere die Verwendung dieser Verbindungen zur Herstellung von pharmazeutischen Präparaten zur Anwendung als gag-Protease-Hemmer, wobei Verbindungen der Formel

$R^2$ - AAN - Q - AAC - $R^1$ (I),

worin AAN ein bivalentes Radikal bestehend aus einem bis fünf bivalenten α-Aminosäureresten bedeutet, welches N-terminal mit dem Rest $R^2$ und C-terminal mit dem Radikal Q verbunden ist, AAC eine Bindung oder ein bivalentes Radikal bestehend aus einem bis fünf bivalenten α-Aminosäureresten bedeutet, welches N-terminal mit dem Radikal Q und C-terminal mit dem Rest $R^1$ verbunden ist, $R^1$ für Hydroxy, verethertes Hydroxy, Amino oder substituiertes Amino mit Ausnahme eines von einer α-Aminosäure abgeleiteten Aminorestes bedeutet, $R^2$ Wasserstoff oder einen Acylrest mit Ausnahme eines gegebenenfalls N-substituierten Acylrestes einer natürlichen Aminosäure bedeutet, und Q einen bivalenten Rest bedeutet, der ein Isoster eines Dipeptides ist, sowie Salze davon verwendet werden.

EP 0 374 097 A2

## Verwendung von Peptidisosteren als retrovirale Proteasehemmer

HIV (human immunodeficiency virus) ist ein Retrovirus, der die im Menschen im allgemeinen tödlich verlaufende Krankheit AIDS verursacht. Während es heute möglich ist, die Auswirkungen von AIDS zu lindern und das Leben der Patienten zu verlängern, fehlen bisher pharmazeutische Präparate, die die Ursache von AIDS wirksam bekämpfen. Eine mögliche Zielrichtung bei der Therapie von AIDS besteht darin, eine Vermehrung von HIV zu verhindern, ohne gleichzeitig die noch intakten Zellverbände des Patienten zu schädigen.

Das Genom der bislang bekannten beiden Arten von HIV, HIV-1 und HIV-2, weist jeweils einen Bereich auf, der eine "gag-Protease" kodiert. Des weiteren enthalten beide Viren einen Bereich, der für die Gruppen-spezifischen Antigene, englisch "group specific antigen" (gag), kodiert. Die entsprechenden Gene werden als Vorläuferprotein exprimiert und daraus proteolytisch von der oben erwähnten gag-Protease die eigentlichen gag-Proteine freigesetzt. Auch die gag-Protease selbst wird aus einem Vorläuferprotein herausgeschnitten, aus dem noch weitere virale Proteine, wie die Reverse Transcriptase und die Integrase, entstehen. Dieser Prozess verläuft vermutlich autoproteolytisch. Es ist ferner bekannt, dass die gag-Protease das "major core protein" (p24) von HIV-1 bzw. HIV-2 vorzugsweise N-terminal bei Prolin spaltet, z.B. bei Phe-Pro, Leu-Pro oder Tyr-Pro. Ein auf diese Weise verkürztes, Pro-terminales p24 dient dann zusammen mit weiteren viralen Strukturproteinen zum Aufbau des Nucleocapsids und der Virushülle von HIV-1 und HIV-2. Des weiteren ist bekannt, dass die HIV-1 gag-Protease Leu-Phe-, Leu-Ala-, Met-Met- und Phe-Leu-Sequenzen mit unterschiedlicher Effizienz spaltet. Es gilt als sicher, dass zusätzlich zur Aminosäuresequenz die Konformation zur Spezifität der gag-Protease beiträgt.

Wenn die Wirkung der gag-Protease unterbunden werden könnte, stünden dem Virus weder funktionelle Strukturproteine noch die notwendigen viralen Enzyme zur Verfügung, und dessen Vermehrung wäre behindert, wenn nicht gar unterbrochen. Es besteht daher ein Bedürfnis für Inhibitoren oder zumindest Hemmer der gag-Protease. Derartigen Verbindungen kommt eine wichtige Rolle als antivirale Mittel gegen AIDS oder andere retrovirale Erkrankungen zu.

Es ist bekannt, dass die gag-Protease von HIV zur Klasse der Aspartatproteinasen gezählt werden kann und von Pepstatin gehemmt wird. Es wurde nun überraschenderweise gefunden, dass Verbindungen vom Typus der Renininhibitoren oder verwandte Aspartatproteinaseinhibitoren als gag-Protease-Inhibitoren geeignet sind, da sie imstande sind, gag-Proteasen bereits im nanomolaren Konzentrationsbereich zu hemmen.

Die Erfindung betrifft daher die Verwendung von Verbindungen vom Typus der Renininhibitoren oder verwandter Aspartatproteinaseinhibitoren als gag-Protease-Inhibitoren. Sie betrifft insbesondere die Verwendung solcher Verbindungen, die gegenüber der gag-Protease als isoliertes Enzym in Konzentrationen von $10^{-6}$ Mol/l oder weniger oder gegenüber der gag-Protease in der Zelle in Konzentrationen von $10^{-5}$ Mol/l oder weniger Hemmwirkungen von 50 % oder mehr zeigen ($IC_{50}$).

Zahlreiche Verbindungen vom Typus der Renininhibitoren sind bereits bekannt. So werden beispielsweise in den Europäischen Patentanmeldungen 143 746, 144 290, 184 550, 236 734, 118 223, 212 903, 45 665, 152 255, 156 322, 173 481, 163 237, 273 696, 161 588, 172 347, 77 028, 104 041, 155 809, 192 554, 209 848, 210 545, 211 580 und 237 202 Renininhibitoren offenbart, deren Verwendung als gag-Protease-Inhibitoren von dieser Erfindung umfasst ist.

Verwandte Aspartatproteinaseinhibitoren sind besipielsweise Inhibitoren von Pepsin, Chymosin, Cathepsin D oder Penicillopepsin.

Die Erfindung betrifft insbesondere die erfindungsgemässe Verwendung von Verbindungen der Formel I,

$R^2$ - AAN - Q - AAC - $R^1$

worin AAN ein bivalentes Radikal bestehend aus einem bis fünf bivalenten $\alpha$-Aminosäureresten bedeutet, welches N-terminal mit dem Rest $R^2$ und C-terminal mit dem Radikal Q verbunden ist, AAC eine Bindung darstellt oder ein bivalentes Radikal bestehend aus einem bis fünf bivalenten $\alpha$-Aminosäureresten bedeutet, welches N-terminal mit dem Radikal Q und C-terminal mit dem Rest $R^1$ verbunden ist, $R^1$ für Hydroxy, verethertes Hydroxy, Amino oder substituiertes Amino mit Ausnahme eines von einer $\alpha$-Aminosäure abgeleiteten Aminorestes steht, $R^2$ Wasserstoff oder einen Acylrest mit Ausnahme eines gegebenenfalls N-substituierten Acylrestes einer natürlichen Aminosäure bedeutet, und Q einen bivalenten Rest bedeutet, der ein Isoster eines Dipeptides ist, ferner die Verwendung von Salzen solcher Verbindungen.

Die Abkürzung AAN steht für den Aminosäureteil, der sich N-terminal vom Dipeptidisosteren Q befindet (amino acid(s) N-terminal). Die Abkürzung AAC steht für den Aminosäureteil, der sich C-terminal vom Dipeptidisosteren Q befindet (amino acid(s) C-terminal). Der C-terminale Aminosäureteil kann auch völlig

fehlen, d.h. AAC stellt dann eine Bindung dar.

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die substituierten $\alpha$-C-Atome der Aminosäurereste, aus denen AAN und AAC bestehen, können die R-, S- oder R,S-Konfiguration haben. Bevorzugt sind Verbindungen der Formel I, worin diese C-Atome die S-Konfiguration aufweisen. Die $\alpha$-Aminosäurereste des Radikals AAN können N-terminal zur Erhöhung der Stabilität der Verbindung der Formel I gegen enzymatischen Abbau durch Niederalkyl, z.B. Methyl oder Ethyl, substituiert sein.

Ein bivalenter $\alpha$-Aminosäurerest, aus denen AAN und AAC bestehen, ist beispielsweise ein Rest einer natürlichen $\alpha$-Aminosäure mit der L-Konfiguration, wie sie normalerweise in Proteinen vorkommen, eines Homologen einer solchen Aminosäure, z.B. worin die Aminosäureseitenkette um eine oder zwei Methylengruppen verlängert oder verkürzt ist und/oder eine Methylgruppe durch Wasserstoff ersetzt ist, einer substituierten aromatischen $\alpha$-Aminosäure, z.B. eines substituierten Phenylalanins oder Phenylglycins, worin der Substituent Niederalkyl, z.B. Methyl, Halogen, z.B. Fluor, Chlor, Brom oder Iod, Hydroxy, Niederalkoxy, z.B. Methoxy, Niederalkanoyloxy, z.B. Acetoxy, Amino, Niederalkylamino, z.B. Methylamino, Diniederalkylamino, z.B. Dimethylamino, Niederalkanoylamino, z.B. Acetylamino oder Pivaloylamino, Niederalkoxycarbonylamino, z.B. tert-Butoxycarbonylamino, Arylmethoxycarbonylamino, z.B. Benzyloxycarbonylamino, und/oder Nitro sein kann und ein- oder mehrfach vorkommt, eines benzanellierten Phenylalanins oder Phenylglycins, wie $\alpha$-Naphthylalanins, oder eines hydrierten Phenylalanins oder Phenylglycins, wie Cyclohexylalanins oder Cyclohexylglycins, einer fünf- oder sechs-gliedrigen cyclischen, benzanellierten $\alpha$-Aminosäure, z.B. Indolin-2-carbonsäure oder 1,2,3,4-Tetrahydroisochinolin-3-carbonsäure, einer natürlichen oder homologen $\alpha$-Aminosäure, in der eine Carboxygruppe der Seitenkette in veresterter oder amidierter Form vorliegt, z.B. als Niederalkylestergruppe, wie Methoxycarbonyl oder tert-Butoxycarbonyl, oder als Carbamoyl-, als Niederalkylcarbamoyl-, wie Methylcarbamoyl, oder als Diniederalkylcarbamoylgruppe, wie Dimethylcarbamoyl, in der eine Aminogruppe der Seitenkette in acylierter Form vorliegt, z.B. als Niederalkanoylamino-, wie Acetylamino oder Pivaloylamino, als Niederalkoxycarbonylamino-, wie tert-Butoxycarbonylamino, oder als Arylmethoxycarbonylaminogruppe, wie Benzyloxycarbonylamino, oder in der eine Hydroxygruppe der Seitenkette in veretherter oder veresterter Form vorliegt, z.B. als Niederalkoxygruppe, wie Methoxy, als Arylniederalkoxygruppe, wie Benzyloxy, oder als Niederalkanoyloxygruppe, wie Acetoxy, oder eines Epimeren einer solchen Aminosäure, d.h. mit der unnatürlichen D-Konfiguration.

Solche Aminosäuren sind beispielsweise Glycin (H-Gly-OH), Alanin (H-Ala-OH), Valin (H-Val-OH), Norvalin ($\alpha$-Aminovaleriansäure), Leucin, (H-Leu-OH), Isoleucin (H-Ile-OH), Norleucin ($\alpha$-Aminohexansäure, H-Nle-OH), Serin (H-Ser-OH), Homoserin ($\alpha$-Amino-$\gamma$-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Nitrophenylalanin, 4-Aminophenylalanin, 4-Chlorphenylalanin, $\beta$-Phenylserin ($\beta$-Hydroxyphenylalanin), Phenylglycin, $\alpha$-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutaminsäure-mono-tert-butylester, Glutamin (H-Gln-OH), $N^{\delta}$-Dimethylglutamin, Histidin (H-His-OH), Arginin (H-Arg-OH), Lysin (H-Lys-OH), $N^{\varepsilon}$-tert-Butoxycarbonyl-lysin, $\delta$-Hydroxylysin, Ornithin ($\alpha,\delta$-Diaminovaleriansäure), $N^{\delta}$-Pivaloyl-ornithin, $\alpha,\gamma$-Diaminobuttersäure oder $\alpha,\beta$-Diaminopropionsäure.

Die in der Beschreibung der vorliegenden Erfindung verwendeten allgemeinen Ausdrücke und Bezeichnungen haben vorzugsweise die folgenden Bedeutungen:

Eine veretherte Hydroxygruppe $R^1$ ist vorzugsweise durch solche organische Reste verethert, die unter physiologischen Bedingungen abspaltbar sind und nach der Abspaltung in der betreffenden Konzentration pharmakologisch unbedenkliche Spaltprodukte liefern.

Verethertes Hydroxy $R^1$ ist z.B. Acyloxyniederalkoxy, worin Acyl die Acylgruppe einer gegebenenfalls verzweigten Niederalkancarbonsäure oder der durch gegebenenfalls verzweigtes Niederalkyl monoveresterten Kohlensäure darstellt, z.B. Niederalkanoyloxyniederalkoxy, wie Acetoxymethoxy, 1-Acetoxyethoxy, Pivaloyloxymethoxy oder 1-Pivaloyloxyethoxy, oder Niederalkoxycarbonyloxyniederalkoxy, wie Ethoxycarbonyloxymethoxy, 1-Ethoxycarbonyloxyethoxy, tert-Butoxycarbonyloxymethoxy oder 1-tert-Butoxycarbonyloxyethoxy.

Verethertes Hydroxy $R^1$ ist ferner vorzugsweise Niederalkoxy, z.B. Methoxy oder Ethoxy, Aryloxy, z.B. Phenoxy, oder Arylniederalkoxy, z.B. Benzyloxy.

Substituiertes Amino $R^1$ ist beispielsweise eine Aminogruppe, welche durch einen oder gegebenenfalls

zwei organische Reste, z.B. unsubstituierte oder substituierte, gesättigte oder ungesättigte, aliphatische Kohlenwasserstoffreste mit bis einschliesslich 18, bevorzugt bis einschliesslich 10, C-Atomen oder unsubstituierte oder substituierte, aromatische, heteroaromatische, aromatisch-aliphatische oder heteroaromatischaliphatische Reste mit bis zu 18, bevorzugt bis einschliesslich 10, C-Atomen, substituiert ist.

Ausgeschlossen als substituiertes Amino $R^1$ sind der Rest einer $\alpha$-Aminosäure oder deren N-substituierte, veresterte oder amidierte Derivate.

Ein unsubstituierter oder substituierter, gesättigter oder ungesättigter, aliphatischer Kohlenwasserstoffrest, welcher die Aminogruppe $R^1$ substituiert, ist beispielsweise gegebenenfalls substituiertes Alkyl mit bis zu 10 C-Atomen, Niederalkenyl oder Niederalkinyl mit bis einschliesslich 7 C-Atomen, oder Cycloalkyl oder Cycloalkylniederalkyl mit 4-10 C-Atomen. Diese Reste können durch eine oder mehrere der im Zusammenhang mit nachstehend definiertem Niederalkyl $R^a$ genannten funktionellen Gruppen, sowie durch Sulfo, substituiert sein.

Als Substituenten sind Hydroxy, Niederalkoxy, z.B. Methoxy, Niederalkanoyloxy, z.B. Acetoxy, substituiertes oder unsubstituiertes Phenyloxy, z.B. Carbamoylphenyloxy oder Carbamoyl-hydroxy-phenyloxy, Carboxy, verestertes Carboxy, z.B. Niederalkoxycarbonyl, wie Methoxycarbonyl oder tert-Butoxycarbonyl, oder ein physiologisch spaltbares verestertes Carboxy, z.B. 1-(Niederalkanoyloxy)-niederalkoxycarbonyl, wie Acetoxymethoxycarbonyl, Pivaloyloxymethoxycarbonyl oder 1-Propionyloxyethoxycarbonyl, 1-(Niederalkoxycarbonyloxy)-niederalkoxycarbonyl, wie 1-(Ethoxycarbonyloxy)-ethoxycarbonyl, oder $\alpha$-Amino-niederalkanoyloxymethoxycarbonyl, wie $\alpha$-Aminoacetoxymethoxycarbonyl oder (S)-$\alpha$-Amino-$\beta$-methylbutyryloxymethoxycarbonyl, Carbamoyl, substituiertes oder unsubstituiertes Niederalkylcarbamoyl, z.B. Hydroxyniederalkylcarbamoyl, wie 2-Hydroxyethylcarbamoyl oder Tris-(hydroxymethyl)-methylcarbamoyl, Amino, Niederalkylamino, z.B. Methylamino, Diniederalkylamino, z.B. Dimethylamino, Niederalkoxycarbonylamino, z.B. tert-Butoxycarbonylamino, Guanidino, über ein Stickstoff-Atom gebundenes, gesättigtes fünf- oder sechsgliedriges, gewünschtenfalls durch Oxo substituiertes Heterocyclyl, z.B. 1-Piperidinyl, 4-Morpholinyl oder 2-Oxo-1-pyrrolidinyl, oder Sulfo bevorzugt.

Ein aromatischer oder aromatisch-aliphatischer Rest in einer Gruppe $R^1$ hat vorzugsweise die gleichen wie nachstehend unter Aryl $R^a$ oder $R^b$ oder Arylniederalkyl $R^a$ genannten Bedeutungen und ist beispielsweise Phenyl oder Phenylniederalkyl.

Diese Reste können im Aromaten z.B. durch Niederalkyl, z.B. Methyl oder Ethyl, Hydroxy, verethertes Hydroxy, z.B. Niederalkoxy, wie Methoxy oder tert-Butoxy, verestertes Hydroxy, z.B. Niederalkanoyloxy, wie Acetoxy, oder Halogen, z.B. Fluor oder Chlor, Carboxy, verestertes Carboxy, z.B. Niederalkoxycarbonyl, wie tert-Butoxycarbonyl, Carbamoyl, Amino, Niederalkylamino, z.B. Methylamino, Diniederalkylamino, z.B. Dimethylamino, acyliertes Amino, z.B. Niederalkoxycarbonylamino, wie tert-Butoxycarbonylamino, sowie durch Nitro substituiert sein.

Niederalkyl in einem Rest Phenylniederalkyl kann durch die gleichen Substituenten wie Alkyl in einem Rest $R^1$ substituiert sein.

Ein heteroaromatischer oder heteroaromatisch-aliphatischer Rest in einer Gruppe $R^1$ hat vorzugsweise die gleichen wie nachstehend unter Heteroaryl $R^a$ und $R^b$ oder Heteroarylniederalkyl $R^a$ genannten Bedeutungen und ist beispielsweise Pyridylniederalkyl, z.B. 2-, 3- oder 4-Pyridylmethyl, Imidazolylniederalkyl, z.B. 2-(4-Imidazolyl)-ethyl oder auch 2-(2-[4-Imidazolyl]-ethylamino)-ethyl, oder Indolylniederalkyl, z.B. 3-Indolylmethyl oder 2-(3-Indolyl)-ethyl.

Substituiertes Amino $R^1$ ist vorzugsweise Alkylamino, z.B. Methyl-, Ethyl-, n-Propyl-, Isopropyl-, n-Butyl-, Isobutyl-, tert-Butyl-, n-Pentyl-, Isopentyl-, n-Hexyl-, n-Octyl- oder n-Decylamino, Diniederalkyl amino, z.B. Dimethylamino oder Diethylamino, Hydroxyniederalkylamino, z.B. 2-Hydroxyethylamino, 1-Hydroxybut-2-ylamino, 5-Hydroxypentylamino oder Tris(hydroxymethyl)-methylamino, Di-(hydroxyniederalkyl)-amino, z.B. Di-(2-hydroxyethyl)-amino, Niederalkoxyniederalkylamino, z.B. 2-Methoxyethylamino, Niederalkanoyloxyniederalkylamino, z.B. 2-Acetoxyethylamino, Phenoxyniederalkylamino oder Phenoxy-hydroxyniederalkylamino, worin Phenoxy gegebenenfalls durch Niederalkyl, Niederalkoxy, Hydroxy, Carboxy, Niederalkoxycarbonyl oder Carbamoyl substituiert ist, z.B. 2-Phenoxyethylamino, 2-(3-Carbamoyl-4-hydroxyphenoxy)-ethylamino oder 3-(3-Carbamoylphenoxy)-2-hydroxy-propylamino, Carboxyalkylamino oder Amino-carboxy-alkylamino, worin der Carboxyrest nicht in 1-Stellung des Alkylrests steht, z.B. 4-Carboxy-n-butylamino, 5-Carboxy-n-pentylamino, 5-Amino-5-carboxy-n-pentylamino, 6-Carboxy-n-hexylamino, 7-Carboxy-n-heptylamino oder 8-Carboxy-n-octylamino, ferner Dicarboxymethylamino, Niederalkoxycarbonylalkylamino oder Acylamino-niederalkoxycarbonyl-alkylamino, worin der Carbonylrest nicht in 1-Stellung des Alkylrests steht, z.B. 4-tert-Butoxycarbonyl-n-butylamino, 5-tert-Butoxycarbonylamino-5-methoxycarbonyl-n-pentylamino, 7-tert-Butoxycarbonyl-n-heptylamino oder 8-tert-Butoxycarbonyl-n-octylamino, ferner Diniederalkoxycarbonyl-methylamino, z.B. Di-methoxycarbonyl-methylamino, physiologisch spaltbares verestertes Carboxyalkylamino, worin die Esterfunktion nicht in 1-Stellung des Alkylrests steht, z.B. 4-Pivaloyloxymethoxycarbonyl-n-butylamino, 7-(1-Ethoxycarbonyloxyethoxycarbonyl)-n-heptylamino oder 7-Pivaloyloxymethoxycarbonyl-n-heptylamino, Carbamoylalkylamino oder Hydroxyniederalkylcarbamoylalkylamino, worin der Carbamoylrest nicht in 1-Stellung des Alkylrests steht, z.B. 4-Carbamoyl-n-butylamino, 7-Carbamoyl-n-heptylamino oder 4-(Tris[hydroxymethyl]-methyl)-carbamoyl-n-butylamino, ferner Dicarbamoylmethylamino, Di-(niederalkylcarbamoyl)-methylamino, z.B. Di-(methylcarbamoyl)-methylamino, Di-(hydroxyniederalkylcarbamoyl)-methylamino, z.B. Di-(2-hydroxyethylcarbamoyl)-methylamino, oder Bis-(diniederalkylcarbamoyl)-methylamino, z.B. Bis-(dimethylcarbamoyl)-methylamino, Aminoniederalkylamino, z.B. 2-Aminoethylamino oder 4-Aminobutylamino, Niederalkylaminoniederalkylamino, z.B. 2-Methylaminoethylamino, Diniederalkylaminoniederalkylamino, z.B. 2-Dimethylaminoethylamino oder 3-Dimethylaminopropylamino, Niederalkoxycarbonylaminoniederalkylamino, z.B. 2-(tert-Butoxycarbonylamino)- ethylamino, Guanidinoniederalkylamino, z.B. 2-Guanidinoethylamino, über ein Stickstoff-Atom gebundenes, gesättigtes fünf- oder sechsgliedriges Heterocyclylniederalkylamino, z.B. 2-(4-Morpholinyl)-ethylamino, 3-(4-Morpholinyl)-propylamino oder 3-(2-Oxo-1-pyrrolidinyl)-propylamino, Niederalkenylamino, z.B. Allylamino oder 2- oder 3-Butenylamino, Niederalkinylamino, z.B. Propargylamino, Cycloalkylamino, z.B. Cyclohexylamino oder Decahydronaphthylamino, Cycloalkylniederalkylamino, z.B. Cyclo-propylmethylamino oder Cyclohexylmethylamino, Naphthylamino, Phenylamino oder Phenylniederalkylamino, worin Phenyl oder Naphthyl gegebenenfalls durch Niederalkyl, z.B. Methyl, Phenyl, Hydroxy, Niederalkoxy, z.B. Methoxy oder tert-Butoxy, Niederalkanoyloxy, z.B. Acetoxy, Halogen, z.B. Fluor oder Chlor, Carboxy, Niederalkoxycarbonyl, z.B. tert-Butoxycarbonyl, Carbamoyl, Amino, Niederalkylamino, z.B. Methylamino, Diniederalkylamino, z.B. Dimethylamino, Acylamino, z.B. tert-Butoxycarbonylamino und/oder durch Nitro mono- oder mehrfach substituiert ist, z.B. Phenylamino, 2-, 3- oder 4-Methylphenylamino, 4-Biphenylylamino, 4-Hydroxyphenylamino, 4-Methoxyphenylamino, 2,3-, 2,4- oder 2,5-Dimethoxyphenylamino, 4-Chlorphenylamino, 2-, 3- oder 4-Carboxyphenylamino, 2-, 3- oder 4-Methoxy- oder tert-Butoxy-carbonylphenylamino, 2-, 3- oder 4-Carbamoylphenylamino, 4-Aminophenylamino, 4-tert-Butoxycarbonylaminophenylamino oder 4-Nitrophenylamino, ferner z.B. Benzylamino, 4-Methylbenzylamino, 4-Biphenylylmethylamino, 4-Methoxybenzylamino, 2-, 3- oder 4-Carboxybenzylamino, 2-, 3- oder 4-tert-Butoxycarbonylbenzylamino, 2-, 3- oder 4-Carbamoylbenzylamino, 2-Phenylethylamino, 3-Phenylpropylamino oder 5-Phenylpentylamino, Pyridylniederalkylamino, z.B. 2-, 3- oder 4-Pyridylmethylamino, 2-(2-, 3- oder 4-Pyridyl)-ethylamino oder 3-(2-, 3- oder 4-Pyridyl)-propylamino, Imidazolylniederalkylamino, z.B. 4-Imidazolylmethylamino, 2-(4-Imidazolyl)-ethylamino oder 2(2-[4-Imidazolyl]-ethylamino)-ethylamino, Indolylniederalkylamino, z.B. 3-Indolylmethylamino oder 2-(3-Indolyl)-ethylamino, oder Sulfoniederalkylamino, z.B. 2-Sulfoethylamino.

Acyl $R^2$ hat z.B. bis zu 25, bevorzugt bis zu 19 C-Atome und ist in erster Linie die Acylgruppe einer Carbonsäure, eines Halbesters der Kohlensäure, einer gegebenenfalls N-substituierten Carbamin- oder Thiocarbaminsäure, eines gegebenenfalls N-substituierten Oxalamids, einer Phosphorsäure, einer Sulfonsäure oder einer gegebenenfalls N-substituierten Amidosulfonsäure.

Bevorzugte Acylgruppen $R^2$ sind beispielsweise Alkanoyl, z.B. n-Decanoyl oder Niederalkanoyl, z.B. Formyl, Acetyl, Propionyl oder Pivaloyl, Hydroxyniederalkanoyl, z.B. $\beta$-Hydroxypropionyl, Niederalkoxyniederalkanoyl, z.B. Niederalkoxyacetyl oder Niederalkoxypropionyl, wie Methoxyacetyl oder $\beta$-Methoxypropionyl, Phenoxyniederalkanoyl, z.B. Phenoxyacetyl, Naphthoxyniederalkanoyl, z.B. $\alpha$- oder $\beta$-Naphthoxyacetyl, Niederalkanoyloxyniederalkanoyl, z.B. Niederalkanoyloxyacetyl oder Niederalkanoyloxypropionyl, wie Acetoxyacetyl oder $\beta$-Acetoxypropionyl, Halogenniederalkanoyl, z.B. $\alpha$-Halogenacetyl, wie $\alpha$-Chlor-, $\alpha$-Brom-, $\alpha$-Iod- oder $\alpha,\alpha,\alpha$-Trichloracetyl, oder Halogenpropionyl, wie $\beta$-Chlor-oder $\beta$-Brompropionyl, Carboxyniederalkanoyl, z.B. Carboxyacetyl oder $\beta$-Carboxypropionyl, Niederalkoxycarbonylniederalkanoyl, z.B. Niederalkoxycarbonylacetyl oder Niederalkoxycarbonylpropionyl, wie Methoxycarbonylacetyl, $\beta$-Methoxycarbonylpropionyl, Ethoxycarbonylacetyl oder $\beta$-Ethoxycarbonylpropionyl, Carbamoylniederalkanoyl, z.B. Carbamoylacetyl oder $\beta$-Carbamoylpropionyl, Niederalkylcarbamoylniederalkanoyl, z.B. Methylcarbamoylacetyl, Diniederalkylcarbamoylniederalkanoyl, z.B. Dimethylcarbamoylacetyl, Oxoniederalkanoyl, z.B. Acetoacetyl oder Propionylacetyl, Hydroxy-carboxy-niederalkanoyl, z.B. $\alpha$-Hydroxy-$\alpha$-carboxy-acetyl oder $\alpha$-Hydroxy-$\beta$-carboxypropionyl, Hydroxy-niederalkoxycarbonyl-niederalkanoyl, z.B. $\alpha$-Hydroxy-$\alpha$-ethoxy- oder -methoxycarbonylacetyl oder $\alpha$-Hydroxy-$\beta$-ethoxy- oder -methoxycarbonyl-propionyl, verestertes Hydroxy-niederalkoxycarbonyl-niederalkanoyl, z.B. $\alpha$-Acetoxy-$\alpha$-methoxycarbonyl-acetyl, Dihydroxy-carboxy-niederalkanoyl, z.B. $\alpha,\beta$-Dihydroxy-$\beta$-carboxy-propionyl, Dihydroxy-niederalkoxycarbonyl-niederalkanoyl, z.B. $\alpha,\beta$-Dihydroxy-$\beta$-ethoxy- oder -methoxycarbonyl-propionyl, verestertes Dihydroxy-niederalkoxycarbonyl-niederalkanoyl, z.B. $\alpha,\beta$-Diacetoxy-$\beta$-methoxycarbonyl-propionyl, $\alpha$-Naphthoxy-carboxy-niederalkanoyl, z.B. 2-$\alpha$-Naphthoxy-4-carboxy-butyryl, $\alpha$-Naphthoxy-niederalkoxycarbonylniederalkanoyl, z.B. $\alpha$-Naphthoxy-ethoxycarbonyl-acetyl, 2-$\alpha$-Naphthoxy-3-ethoxycarbonyl-propionyl oder 2-$\alpha$-Naphthoxy-4-tert-butoxycarbonylbutyryl, $\alpha$-Naphthoxy-benzyloxycarbonyl-niederalkanoyl, z.B. 2-$\alpha$-Naph thoxy-3-benzyloxycarbonyl-propionyl, $\alpha$-Naphthoxy-carbamoyl-niederealkanoyl, z.B. 2-$\alpha$-Naphthoxy-4-carbamoyl-butyryl, $\alpha$-Naphthoxy-cy-

anoniederalkanoyl, z.B. α-Naphthoxy-cyano-acetyl oder 2-α-Naphthoxy-4-cyanobutyryl, α-Naphthoxy-diniederalkylamino-niederalkanoyl, z.B. 2-α-Naphthoxy-5-dimethylamino-pentanoyl, α-Naphthoxy-oxo-niederalkanoyl, z.B. 2-α-Naphthoxy-4-oxo-pentanoyl, Heterocyclylniederalkanoyl, z.B. Indolylacetyl oder Benzofuranylacetyl, Niederalkenoyl, z.B. Acryloyl, Vinylacetyl, Crotonoyl oder 3- oder 4-Pentenoyl, Niederalkinoyl, z.B. Propioloyl oder 2- oder 3-Butinoyl, Cycloalkylcarbonyl, z.B. Cyclopropyl-, Cyclobutyl-, Cyclopentyl- oder Cyclohexylcarbonyl, Bicycloalkylcarbonyl, z.B. Decahydronaphthyl-2-carbonyl, endo- oder exo-Norbornyl-2-carbonyl, Bicyclo[2.2.2]oct-2-ylcarbonyl oder Bicyclo[3.3.1]non-9-ylcarbonyl, Tricycloalkylcarbonyl, z.B. 1- oder 2-Adamantylcarbonyl, Cycloalkenylcarbonyl, z.B. 1-Cyclohexenylcarbonyl oder 1,4-Cyclohexadienylcarbonyl, Bicycloalkenylcarbonyl, z.B. 5-Norbornen-2-ylcarbonyl oder Bicyclo[2.2.2]octen-2-ylcarbonyl, Cycloalkylniederalkanoyl, z.B. Cyclopropylacetyl, Cyclopentylacetyl, Cyclohexylacetyl oder 3-Cyclohexylpropionyl, Cycloalkylniederalkenoyl, z.B. Cyclohexylacryloyl, Cycloalkenylniederalkanoyl, z.B. 1-Cyclohexenylacetyl oder 1,4-Cyclohexadienylacetyl, Benzoyl, unsubstituiert, mono- oder mehrfachsubstituiert durch Niederalkyl, z.B. Methyl, Phenyl, Halogen, z.B. Chlor, Hydroxy, Niederalkoxy, z.B. Methoxy, und/oder Nitro, z.B. 4-Chlor-, 4-Methoxy- oder 4-Nitrobenzoyl, ferner Phenyl-, α-Naphthyl- oder β-Naphthylniederalkanoyl, worin Phenyl unsubstituiert, mono- oder mehrfachsubstituiert durch Niederalkyl, z.B. Methyl, Phenyl, Halogen, z.B. Chlor, Hydroxy, Niederalkoxy, z.B. Methoxy, und/oder Nitro und Niederalkanoyl unsubstituiert oder substituiert z.B. durch Hydroxy, Niederalkoxy, Acyloxy, Carboxy, verestertes Carboxy, Carbamoyl, substituiertes Carbamoyl, Cyano, Phosphono, verestertes Phosphono, Benzofuranyl und/oder Oxo sein kann und gegebenenfalls verzweigt ist, z.B. Phenylacetyl, α-Naphthylacetyl, β-Naphthylacetyl, Niederalkylphenylacetyl, wie 4-Methylphenylacetyl, Niederalkoxyphenylacetyl, wie 4-Methoxyphenylacetyl, 3-Phenylpropionyl, 3-(p-Hydroxyphenyl)-propionyl, Diphenylacetyl, Di-(4-methoxyphenyl)-acetyl, Triphenylacetyl, substituiertes Anilinophenylacetyl, wie 2-(o,o-Dichloranilino)-phenylacetyl oder 2-(o,o-Dichloro-N-benzylanilino)-phenylacetyl, 3-α- oder β-Naphthyl propionyl, 3-Phenyl- oder 3-α-Naphthyl-2-hydroxy-propionyl, 3-Phenyl-oder 3-α-Naphthyl-2-niederalkoxy-propionyl, wie 3-Phenyl- oder 3-α-Naphthyl-2-neopentyloxy-propionyl, 3-Phenyl- oder 3-α-Naphthyl-2-acyloxy-propionyl, wie 3-Phenyl-2-pivaloyloxy- oder 2-acetoxy-propionyl, 3-α-Naphthyl-2-pivaloyloxy- oder 2-acetoxy-propionyl, 3-α-Naphthyl-2-acetoacetoxy-propionyl, 3-α-Naphthyl-2-ethylaminocarbonyloxy-propionyl oder 3-α-Naphthyl-2-(2-amino- oder 2-benzyloxycarbonylamino-2-methylpropionyloxy)-propionyl, 3-Phenyl- oder 3-α-Naphthyl-2-carboxymethyl-propionyl, 3-Phenyl- oder 3-α-Naphthyl-2-niederalkoxycarbonyl-propionyl, wie 3-α-Naphthyl-2-ethoxycarbonyl-propionyl, 3-Phenyl- oder 3-α-Naphthyl-2-benzyloxycarbonylmethyl-propionyl, 3-Phenyl- oder 3-α-Naphthyl-2-carbamoyl-propionyl, 3-Phenyl- oder 3-α-Naphthyl-2-tert-butylcarba moyl-propionyl, 3-Phenyl- oder 3-α-Naphthyl-2-(2-dimethylaminoethyl)-carbamoyl-propionyl, 3-α-Naphthyl-2-(carboxy- oder tert-butoxycarbonyl)-methylcarbamoyl-propionyl, 3-Phenyl- oder 3-α-Naphthyl-2-(3-hydroxy-2-propyl)-carbamoyl-propionyl, 3-Phenyl- oder 3-α-Naphthyl-2-(2,2-dimethoxyethyl)-carbamoyl-propionyl, 3-Phenyl- oder 3-α-Naphthyl-2-(5-amino-5-carboxypentyl)-carbamoyl-propionyl, 3-Phenyl- oder 3-α-Naphthyl-2-cyano-propionyl, 3-Phenyl- oder 3-α-Naphthyl-2-cyanomethyl-propionyl, 3-Phenyl-2-phosphono- oder phosphonomethyl-propionyl, 3-Phenyl-2-dimethoxyphosphoryl- oder dimethoxyphosphorylmethyl-propionyl, 3-Phenyl-2-diethoxyphosphoryl- oder diethoxyphosphorylmethyl-propionyl, 3-Phenyl-2-ethoxy- oder methoxy-hydroxyphosphoryl-propionyl, 3-Phenyl- oder 3-α-Naphthyl-2-acetonyl-propionyl, 3-Phenyl- oder 3-α-Naphthyl-2-dimethylaminomethyl-propionyl, 2-Benzyl- oder 2-α-Naphthylmethyl-4-cyano-butyryl, 4-Phenyl- oder 4-α-Naphthyl-3-carboxy-butyryl, 4-Phenyl- oder 4-α-Naphthyl-3-benzyloxycarbonyl-butyryl, 2-Benzyl-4-(2-benzofuranyl)-4-oxobutyryl, 2-Benzyl- oder 2-α-Naphthylmethyl-4-oxo-pentanoyl, 2-Benzyl-oder 2-α-Naphthylmethyl-4,4-dimethyl-3-oxo-pentanoyl, 2-Benzyl- oder 2-α-Naphthylmethyl-5-dimethylamino-pentanoyl, 2-Benzyl- oder 2-α-Naphthylmethyl-5-dimethylamino-4-oxo-pentanoyl, 2-Benzyl- oder 2-α-Naphthylmethyl-5,5-dimethyl-4-oxo-hexanoyl, α,p-Diamino-phenylacetyl, α,p-Diacylamino-phenylacetyl, wie α,p-Dibenzyloxycarbonylamino-phenylacetyl oder α-Pivaloylamino-p-benzyloxycarbonylamino-phenylacetyl, Phenylniederalkenoyl, z.B. β-Phenylacryloyl oder β-Phenylvinylacetyl, Naphthylcarbonyl, z.B. α- oder β-Naphthylcarbonyl oder 1,8-Naphthalindicarbonyl, Indenylcarbonyl, z.B. 1-, 2- oder 3-Indenylcarbonyl, Indanylcarbonyl, z.B. 1- oder 2-Indanylcarbonyl, Phenanthrenylcarbonyl, z.B. 9-Phenanthrenylcarbonyl, gegebenenfalls substituiertes Pyrrolylcarbonyl, z.B. 2- oder 3-Pyrrolylcarbonyl oder 4- oder 5-Phenylpyrrolyl-2-carbonyl, Furylcarbonyl, z.B. 2-Furylcarbonyl, Thienylcarbonyl, z.B. 2-Thienylcarbonyl, Pyridylcarbonyl, z.B. 2-, 3- oder 4-Pyridylcarbonyl, gegebenenfalls substituiertes Indolylcarbonyl, z.B. 2-, 3- oder 5-Indolylcarbonyl, 1-Methyl-, 5-Methyl-, 5-Methoxy-, 5-Benzyloxy-, 5-Chlor- oder 4,5-Dimethylindolyl-2-carbonyl, 1-Benzylindolyl-2- oder 3-carbonyl, 4,5,6,7-Tetrahydroindolyl-2-carbonyl, Cyclohepta[b]pyrrolyl-5-carbonyl, gegebenenfalls substituiertes Chinolylcarbonyl, z.B. 2-, 3- oder 4-Chinolylcarbonyl oder 4-Hydroxychinolyl-2-carbonyl, gegebenenfalls substituiertes Isochinolylcarbonyl, z.B. 1-, 3- oder 4-Isochinolylcarbonyl oder 1-Oxo-1,2-dihydroisochinolyl-3-carbonyl, 2-Chinoxalinylcarbonyl, 2-Benzofuranylcarbonyl, 3-Chromancarbonyl, 3-Thiochromancarbonyl, Benz[e]indolyl-2-carbonyl, β-Carbolinyl-3-carbonyl, Pyrrolidinyl-3-carbonyl, Hydroxypyrrolidinylcarbonyl, z.B. 3- oder 4-Hydroxypyrrolidinyl-2-carbonyl, Oxopyrrolidinylcarbonyl, z.B. 5-Oxopyrrolidinyl-2-carbonyl, Piperidinylcarbonyl, z.B. 2-, 3- oder 4-Piperidinylcarbonyl, Indolinylcarbonyl, z.B. 2- oder 3-Indolinylcarbonyl, 1,2,3,4-Tetrahydrochinolylcarbonyl, z.B. 1,2,3,4-Tetrahydrochinolyl-2-, -3- oder -4-carbonyl, 1,2,3,4-Tetrahydroisochinolylcarbonyl, z.B. 1,2,3,4-Tetrahydroisochinolyl-1-, -3- oder 4-carbonyl oder 1-Oxo-1,2,3,4-tetrahydroisochinolyl-3-carbonyl, Niederalkoxycarbonyl, z.B. Methoxy-, Ethoxy-oder tert-Niederalkoxycarbonyl, wie tert-Butoxycarbonyl, 2-Halogenniederalkoxycarbonyl, z.B. 2-Chlor-, 2-Brom-, 2-Iod- oder 2,2,2-Trichlorethoxycarbonyl, Arylniederalkoxycarbonyl, z.B. Arylmethoxycarbonyl, worin Aryl Phenyl, Biphenylyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Chlor oder Brom, und/oder Nitro mono- oder mehrfachsubstituiertes Phenyl ist, z.B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl oder Trityloxycarbonyl, Fluorenylniederalkoxycarbonyl, wie 9-Fluorenylmethoxycarbonyl, ferner Oxamoyl oder Niederalkyloxamoyl, z.B. Methyl- oder Ethyloxamoyl.

Acyl $R^2$ ist ferner in erster Linie eine durch eine Thio-, Sulfinyl- oder Sulfonylgruppe und gegebenenfalls durch weitere, Heteroatome enthaltende Gruppen substituierte Acylgruppe einer gesättigten oder ungesättigten, aliphatischen, cycloaliphatischen, cycloaliphatisch-aliphatischen, aromatischen, aromatisch-aliphatischen, heteroaromatischen oder heteroaromatisch-aliphatischen Carbonsäure mit Ausnahme der gegebenenfalls N-substituierten natürlichen Aminosäure Methionin.

Bevorzugte Substituenten $R^2$ sind Acylgruppen der Formel

$$R^a - \underset{(O)_m}{S} - (CH_2)_n - \underset{\underset{R^b}{\underset{|}{\underset{(CH_2)_q}{|}}}}{CH} - (CH_2)_p - \underset{O}{\overset{}{C}} - \qquad (Ia),$$

worin $R^a$ unsubstituiertes oder substituiertes Niederalkyl, Niederalkenyl, Niederalkinyl, Mono-, Bi- oder Tricycloalkyl, Cycloalkylniederalkyl, unsubstituiertes oder substituiertes Aryl, Arylniederalkyl, Arylniederalkenyl, unsubstituiertes oder substituiertes Heteroaryl, Heteroarylniederalkyl, unsubstituiertes oder substituiertes Hydroxy oder unsubstituiertes oder substituiertes Amino, $R^b$ Wasserstoff, Mono-, Bi-oder Tricycloalkyl, unsubstituiertes oder substituiertes Aryl oder unsubstituiertes oder substituiertes Heteroaryl, m 0, 1 oder 2, n 0, 1 oder 2, p 0, 1 oder 2 und q 0, 1, 2, 3 oder 4 darstellt.

Das Methin-Kohlenstoffatom in der Teilformel Ia und, falls m 1 ist, auch das Schwefelatom können in der R-, S- oder R,S-Konfiguration vorliegen.

Niederalkyl $R^a$ hat vorzugsweise 1 - 7 C-Atome und ist z.B. Methyl, Ethyl, n-Propyl, Isopropyl, n-Butyl oder tert-Butyl, welche durch eine oder mehrere funktionelle Gruppen, beispielsweise Hydroxy, verethertes Hydroxy, z.B. Niederalkoxy, wie Methoxy oder Ethoxy, oder Phenyloxy, verestertes Hydroxy, z.B. Niederalkanoyloxy, wie Acetoxy, Halogen, z.B. Chlor oder Brom, Hydroxysulfonyloxy, Carboxy, verestertes Carboxy, z.B. Niederalkoxycarbonyl, wie Methoxy- oder Ethoxycarbonyl, amidiertes Carboxy, z.B. Carbamoyl oder Mono- oder Diniederalkylcarbamoyl, wie Methyl- oder Dimethylcarbamoyl, Cyano, Amino, substituiertes Amino, z.B. Mononiederalkylamino, Diniederalkylamino, Acylamino oder substituiertes Amino, worin die Aminogruppe Teil eines fünf- oder sechsgliedrigen Heterocyclus enthaltend ein bis zwei Stickstoffatome und gewünschtenfalls ein Sauerstoff- oder Schwefelatom ist, oder durch Oxo substituiert sein können.

Substituiertes Niederalkyl $R^a$ ist beispielsweise Hydroxyniederalkyl, z.B. 2-Hydroxyethyl, Niederalkoxyniederalkyl, z.B. Niederalkoxyethyl, wie 2-Methoxyethyl, Phenoxyniederalkyl, z.B. 2-Phenoxyethyl, Niederalkanoyloxyniederalkyl, z.B. Niederalkanoyloxyethyl, wie 2-Acetoxyethyl, Halogenniederalkyl, z.B. Halogenethyl, wie 2-Chlor- oder 2-Bromethyl, Hydroxysulfonyloxyniederalkyl, z.B. 2-Hydroxysulfonyloxyethyl, Carboxyniederalkyl, z.B. Carboxymethyl oder 2-Carboxyethyl, Niederalkoxycarbonylniederalkyl, z.B. Niederalkoxycarbonylmethyl oder Niederalkoxycarbonylethyl, wie Methoxycarbonylmethyl, 2-Methoxycarbonylethyl, Ethoxycarbonylmethyl oder 2-Ethoxycarbonylethyl, Carbamoylniederalkyl, z.B. Carbamoylmethyl oder 2-Carbamoylethyl, Niederalkylcarbamoylniederalkyl, z.B. Methylcarbamoylmethyl, Diniederalkylcarbamoylniederalkyl, z.B. Dimethylcarbamoylmethyl, Cyanoniederalkyl, z.B. 2-Cyanoethyl, Aminoniederalkyl, z.B. 2-Aminoethyl, Niederalkylaminoniederalkyl, z.B. 2-Methylaminoethyl, Diniederalkylaminoniederalkyl, z.B. 2-Dimethylaminoethyl, Morpholinoniederalkyl, z.B. 2-Morpholinoethyl, Piperidinoniederalkyl, z.B. 2-Piperidinoethyl, Acylaminoniederalkyl, z.B. Niederalkanoylaminoniederalkyl, wie 2-Acetylaminoethyl, Benzyloxycarbonylaminoniederalkyl, wie 2-Benzyloxycarbonylaminoethyl, Niederalkoxycarbonylaminoniederalkyl, wie 2-tert-Butoxycarbonylaminoethyl, oder Oxoniederalkyl, z.B. 2-Oxopropyl oder 2-Oxobutyl.

Niederalkenyl $R^a$ enthält z.B. 2 - 7, insbesondere 2 - 4, C-Atome und ist z.B. Vinyl, Allyl oder 2- oder 3-Butenyl. Niederalkenyl $R^a$ kann durch die gleichen Substituenten substituiert sein wie Niederalkyl, z.B. durch Hydroxy, verethertes Hydroxy, z.B. Methoxy, verestertes Hydroxy, z.B. Acetoxy, Halogen, z.B. Chlor oder Brom, Carboxy, verestertes Carboxy, z.B. Methoxycarbonyl oder Ethoxycarbonyl, oder amidiertes Carboxy, z.B. Carbamoyl.

Niederalkinyl $R^a$ enthält z.B. 2 - 7, insbesondere 2 - 4, C-Atome und ist beispielsweise Ethinyl, 1-Propinyl oder 2-Propinyl.

Cycloalkyl $R^a$ oder $R^b$ enthält z.B. 3 - 8, insbesondere 3 - 6, C-Atome und ist z.B. Cyclopropyl, Cyclobutyl, Cyclopentyl oder Cyclohexyl.

Bicycloalkyl $R^a$ oder $R^b$ enthält z.B. 5 - 10, insbesondere 6 - 9, C-Atome und ist z.B. Bicyclohexyl, -heptyl, -octyl, -nonyl oder -decyl, z.B. Bicyclo[3.1.0]hex-1-, -2- oder -3-yl, Bicyclo[4.1.0]hept-1- oder -7-yl, Bicyclo[2.2.1]hept-2-yl, z.B. endo- oder exo-Norbornyl, Bicyclo[3.2.1]oct-2-yl, Bicyclo[3.3.0]oct-3-yl oder Bicyclo[3.3.1]non-9-yl, ferner α- oder β-Decahydronaphthyl.

Tricycloalkyl $R^a$ oder $R^b$ enthält z.B. 8 - 10 C-Atome und ist z.B. Tricyclo[5.2.1.0$^{2,6}$]dec-8-yl oder Adamantyl, wie 1-Adamantyl.

Cycloalkylniederalkyl $R^a$ enthält z.B. 4 - 10, insbesondere 4 - 7, C-Atome und ist beispielsweise Cyclopropylmethyl, Cyclobutylmethyl, Cyclopentylmethyl oder Cyclohexylmethyl.

Die genannten cycloaliphatischen oder cycloaliphatisch-aliphatischen Reste können durch die gleichen Substituenten wie Niederalkyl $R^a$ substituiert sein.

Aryl $R^a$ oder $R^b$ enthält z.B. 6 bis 14 C-Atome und ist beispielsweise Phenyl, Indenyl, z.B. 2- oder 4-Indenyl, 1- oder 2-Naphthyl, Anthryl, z.B. 1- oder 2-Anthryl, Phenanthryl, z.B. 9-Phenanthryl, oder Acenaphthenyl, z.B. 1-Acenaphthenyl. Aryl $R^a$ oder $R^b$ ist beispielsweise durch Niederalkyl, z.B. Methyl, Phenyl, Hydroxy, Niederalkoxy, z.B. Methoxy, Acyloxy, z.B. Niederalkanoyloxy, wie Acetoxy, Amino, Niederalkylamino, z.B. Methylamino, Diniederalkylamino, z.B. Dimethylamino, Acylamino, z.B. tert-Butoxycarbonylamino, oder Halo, z.B. Chlor, Brom oder Iod, substituiert, wobei der Substituent in irgendeiner Stellung des Arylrestes, z.B. in o-, m- oder p-Stellung des Phenylrestes, stehen kann und der Arylrest auch mehrfach mit gleichen oder verschiedenen Substituenten substituiert sein kann.

Arylniederalkyl $R^a$ hat z.B. 7 bis 15 C-Atome und enthält beispielsweise einen unter Niederalkyl $R^a$ genannten unsubstituierten oder substituierten, gegebenenfalls verzweigten Rest und einen unter Aryl $R^a$ oder $R^b$ genannten unsubstituierten oder substituierten Rest. Ein solches Arylniederalkyl ist beispielsweise Benzyl, Niederalkylbenzyl, wie 4-Methylbenzyl, Biphenylylmethyl, wie 4-Biphenylylmethyl, Niederalkoxybenzyl, wie 4-Methoxybenzyl, 2-Phenylethyl, 2-(p-Hydroxyphenyl)-ethyl, Diphenylmethyl, Di-(4-methoxyphenyl)-methyl, Trityl oder α- oder β-Naphthylmethyl.

Arylniederalkenyl $R^a$ hat z.B. 8 bis 16 C-Atome und enthält beispielsweise einen unter Niederalkenyl $R^a$ genannten unsubstituierten oder substituierten Rest und einen unter Aryl $R^a$ oder $R^b$ genannten unsubstituierten oder substituierten Rest. Ein solches Arylniederalkenyl ist beispielsweise Styryl, 3-Phenylallyl, 2-(α-Naphthyl)-vinyl oder 2-(β-Naphthyl)-vinyl.

Unsubstituiertes oder substituiertes Heteroaryl $R^a$ oder $R^b$ ist mono-, bi-oder tricyclisch und enthält ein bis zwei Stickstoffatome und/oder ein Sauerstoff- oder Schwefelatom. $R^a$ oder $R^b$ ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanelliertes, cyclopenta-, cyclohexa- oder cycloheptaanelliertes Derivat dieser Reste. Dieser Heterocyclus kann an einem Stickstoffatom durch Oxido, Niederalkyl, z.B. Methyl oder Ethyl, Phenyl oder Phenylniederalkyl, z.B. Benzyl, und/oder an einem oder mehreren Kohlenstoff-Atomen durch Niederalkyl, z.B. Methyl, Phenyl, Phenylniederalkyl, z.B. Benzyl, Halogen, z.B. Chlor, Hydroxy, Niederalkoxy, z.B. Methoxy, Phenylniederalkoxy, z.B. Benzyloxy, oder Oxo substituiert und teilweise gesättigt sein und ist beispielsweise 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-Pyridinio, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5- Methoxy-, 5-Benzyloxy-, 5-Chlor- oder 4,5-Dimethyl-2-indolyl, 1-Benzyl-2-oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder β-Carbolin-3-yl.

Heteroarylniederalkyl $R^a$ enthält z.B. einen unter Niederalkyl $R^a$ genannten unsubstituierten oder substituierten Rest und einen unter Heteroaryl $R^a$ oder $R^b$ genannten unsubstituierten oder substituierten Rest und ist beispielsweise 2- oder 3-Pyrrolylmethyl, 2-, 3- oder 4-Pyridylmethyl, 2-(2-, 3- oder 4-Pyridyl)-ethyl, 4-Imidazolylmethyl, 2-(4-Imidazolyl)-ethyl, 2- oder 3-Indolylmethyl, 2-(3-Indolyl)-ethyl oder 2-Chinolylmethyl.

Hydroxy $R^a$ ist unsubstituiert oder beispielsweise durch Niederalkyl oder Aryl substituiert und ist z.B. Hydroxy, Methoxy, Ethoxy n-Butoxy, Phenoxy, 4-Hydroxyphenoxy oder 3,4-Methylendioxyphenoxy.

Amino $R^a$ ist unsubstituiert, durch eine oder zwei Niederalkylgruppen oder durch Arylniederalkyl, Niederalkanoyl, Niederalkoxycarbonyl oder Arylmethoxycarbonyl substituiert oder Teil eines fünf- oder sechsgliedrigen Heterocyclus enthaltend ein bis zwei Stickstoffatome und gewünschtenfalls ein Sauerstoff- oder Schwefelatom und ist z.B. Amino, Methylamino, Ethylamino, Isopropylamino, n-Butylamino, Dimethylamino, Diethylamino, Benzylamino, Acetylamino, Pivaloylamino, Methoxy-, Ethoxy- oder tert-Butoxycarbonylamino, Benzyloxycarbonylamino, 1-Pyrrolidinyl, 1-Piperidinyl, 1-Methyl-4-pyridazinyl, 4-Morpholinyl oder 4-Thiomorpholinyl.

Ein bivalenter Rest Q, der für ein Isoster eines Dipeptides steht, ist ein Rest, bei dem die Dipeptidatomfolge der Formel

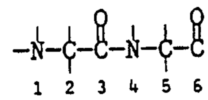

(II)

an beliebiger Stelle so modifiziert ist, dass die Hydrolysierbarkeit der zentralen Amidbindung stark herabgesetzt wird. Stark herabgesetzt im Sinne dieser Erfindung ist die Hydrolysierbarkeit dann, wenn die resul tierende Verbindung der Formel I gegenüber gag-Protease eine Hemmwirkung bei den vorstehend angegebenen Konzentrationsbereichen aufweist. Die Herabsetzung der Hydrolysierbarkeit kann durch Kettenverlängerung auf mehr als sechs Atome, Kettenverkürzung auf weniger als sechs Atome und/oder durch Modifizierung von einem oder mehreren der sechs Atome der Dipeptidatomfolge der Formel II erreicht werden.

Beispiele für geeignete Reste Q geben die folgenden Formeln, in denen die Positionen 3 bis 5 der Formel II, d.h. die Teilstruktur

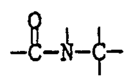

unter Beibehaltung der terminalen Positionen 1 und 2 sowie 6 wie folgt variiert sind:

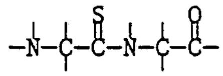 (IIa)

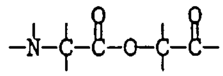 (IIb)

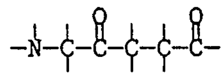 (IIc)

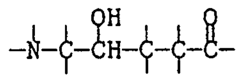 (IId)

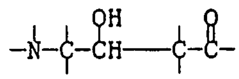 (IIe)

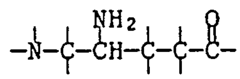 (IIf)

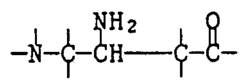 (IIg)

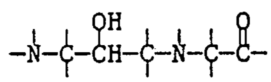 (IIh)

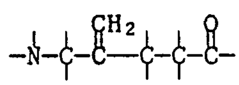 (IIi)

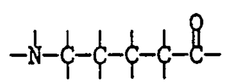 (IIj)

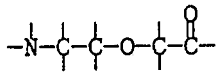 (IIk)

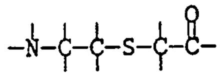 (IIl)

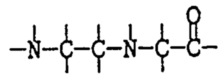 (IIm)

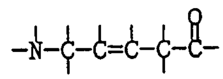 (IIn)

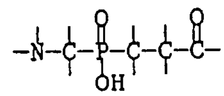 (IIo)

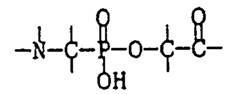 (IIp)

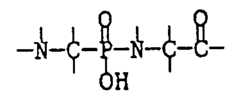 (IIq)

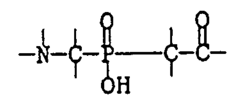 (IIr)

worin freie Valenzen, die nicht an AAN bzw. AAC gebunden sind, unabhängig voneinander durch Wasserstoff oder einen Rest der Art $R^a$, wie vorstehend im Zusammenhang mit $R^2$ definiert, abgesättigt und/oder freie Valenzen an benachbarten N- und C-Atomen durch Niederalkylen, beispielsweise mit 2-5 C-Atomen, überbrückt sind. Derartige Isostere sind bereits grundsätzlich bekannt, z.B. sind sie in den Europäischen Patentanmeldungen 156 322 und 273 696 beschrieben.

Bevorzugte Isostere Q sind solche der Formeln IIa bis IIr, in denen die freien Valenzen, die nicht an AAN bzw. AAC gebunden sind, in einer Weise mit Wasserstoff beziehungsweise mit Resten $R^a$ abgesättigt und/oder durch Niederalkylen überbrückt sind, dass die resultierenden Isostere Dipeptidresten wie Leucin-Valin, Phenylalanin-Valin, Cyclohexylalanin-Valin, Leucin-Alanin, Cyclohexylalanin-Alanin, Leucin-Glycin, Cyclohexylalanin-Glycin, Tyrosin-Prolin, Phenylalanin-Prolin oder Cyclohexylalanin-Prolin entsprechen, insbesondere den Dipeptidfragmenten Leucin-Valin oder Cyclohexylalanin-Valin.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z.B. einer Carboxygruppe, gebildet und sind in erster Linie geeignete Alkalimetall-, z.B. Natrium- oder Kalium-, oder Erdalkalimetallsalze, z.B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, gebildet werden, z.B. mit Diethylamin, Di-(2-hydroxyethyl)-amin, Triethylamin, N,N-Dimethyl-N-(2-hydroxyethyl)-amin, Tri-(2-hydroxyethyl)-amin oder N-Methyl-D-glucamin. Die Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z.B. den weiter vorn genannten α-Aminosäuren, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Verbindungen vom Typus der Renininhibitoren, wie insbesondere Verbindungen der Formel I, weisen gag-Protease-hemmende Wirkungen auf. Insbesondere hemmen sie in Konzentrationen bis herunter zum nanomolaren Bereich die Wirkung von gag-Proteasen von HIV-1 und HIV-2 und sind daher geeignet als Mittel gegen durch Retroviren verursachte Krankheiten, wie z.B. gegen AIDS.

Die Fähigkeit der Verbindungen der Formel I, die proteolytische Aktivität von z.B. HIV-1 Protease zu inhibieren, lässt sich beispielsweise gemäss dem von J. Hansen et al., The EMBO Journal 7, 1785-1791 (1988), beschriebenen Verfahren demonstrieren. Dabei wird die Hemmung der Wirkung der gag-Protease auf ein Substrat gemessen, das ein in E. coli exprimiertes Fusionsprotein aus dem gag-Vorläuferprotein und MS-2 ist. Das Substrat und seine Spaltprodukte werden durch Polyacrylamid-Gelelektrophorese getrennt und durch Immunoblotting mit monoklonalen Antikörpern gegen MS-2 sichtbar gemacht.

In einem noch einfacher zu handhabenden Test, der genaue quantitative Aussagen ermöglicht, wird als Substrat für die gag-Protease ein synthetisches Icosapeptid eingesetzt, das der Spaltstelle des gag-Vorläuferproteins entspricht. Dieses Substrat und seine Spaltprodukte können durch Hochdruckflüssig-Chromatographie (HPLC) gemessen werden. Verbindungen der vorliegenden Erfindung zeigen in diesem Test Hemmwirkungen in Konzentrationen von $10^{-6}$ Mol/l.

In einem weiteren Test kann gezeigt werden, dass die Verbindungen der vorliegenden Erfindung Zellen, die normalerweise von HIV infiziert werden, vor einer solchen Infektion schützt oder zumindest eine solche Infektion verlangsamt. Dabei wird die menschliche T-Zell-Leukämie Zellinie MT-2 (Science 229, 563 (1985)), die extrem empfindlich für den zytopathogenen Effekt von HIV ist, mit HIV allein oder mit HIV in Gegenwart der erfindungsgemässen Verbindungen inkubiert und nach einigen Tagen die Lebensfähigkeit der so behandelten Zellen beurteilt. Erfindungsgemässe Verbindungen zeigen Infektions-hemmende Wirkungen in Konzentrationen von $10^{-5}$ Mol/l.

Spezielle Verbindungen, deren Verwendung als gag-Proteasehemmer bevorzugt ist, können den eingangs genannten europäischen Patentanmeldungen entnommen werden. Die dort genannten Bevorzugungen gelten ebenfalls für die vorliegende Erfindung. Dies gilt in besonderem Masse für die Verbindungen, die in den Europäischen Patentanmeldungen 143 746, 144 290, 184 550 und 236 734 offenbart sind.

Bevorzugte Verbindungen der Formel I sind dadurch gekennzeichnet, dass sie im Radikal AAN zwei oder mehr α-Aminosäurereste aufweisen oder dass das Radikal AAN aus nur einem α-Aminosäurerest besteht und gleichzeitig der Rest $R^2$ ein Analogon zu Phenylalanyl (H-Phe) ist und/oder dass sie im Radikal AAC zwei α-Aminosäurereste aufweisen oder dass das Radikal AAC aus nur einem α-Aminosäurerest besteht und gleichzeitig der Rest $R^1$ ein Analogon zum über Stickstoff gebundenen Rest der Aminosäure Tyrosin (-Tyr-OH) ist.

Ein Rest $R^2$, der als Analogon zu Phenylalanyl gelten kann, weist das Strukturelement der Formel

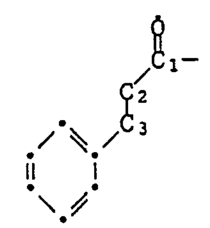

(Ib)

auf, worin der carbocyclische Ring auch ganz oder teilweise gesättigt sein kann, eines der Kohlenstoffatome des carbocyclischen Ringes mit $C_2$ oder $C_3$ zu einem vorzugsweise fünf- oder sechsgliedrigen Ring verknüpft sein kann, eines der Kohlenstoffatome $C_2$ oder $C_3$ durch ein Heteroatom, wie NH, O oder S, ersetzt sein kann, die Carbonylgruppe eine Heterocarbonylgruppe wie P=O sein kann und freie Valenzen Wasserstoff oder Substituenten tragen, beispielsweise Niederalkyl, Niederalkylthioniederalkyl, Niederalkylsulfinylniederalkyl, Niederalkylsulfonylniederalkyl, Phenylniederalkyl, Hydroxy, Niederalkoxy, Phenylniederalkoxy, Aminoniederalkyl, Niederalkylaminoniederalkyl, Niederalkanoylaminoniederalkyl, Niederalkanoyl, Niederalkanoylniederalkyl oder Niederalkylcarbamoyl, wobei diese Substituenten bevorzugt an $C_2$ vorkommen.

Ein ganz oder teilweise gesättigter carbocyclischer Ring ist beispielsweise in diesem Zusammenhang Cyclohexyl oder Cyclohexenyl. Fünf- oder sechsgliedrige Ringe im Sinne des vorstehenden Absatzes sind beispiels weise Phenyl, Cyclohexyl, Pyrrolyl, Pyranyl, Dihydropyranyl oder Thioanaloga der genannten Heterocyclylreste. Diese Definitionen gelten auch für die nachfolgend beschriebenen zu Tyrosin analogen Reste $R^1$.

Derartige Analoga $R^2$ sind beispielsweise 3-Phenyl- oder 3-Cyclohexylpropionyl, die insbesondere in 2-Stellung durch Substituenten der Art Niederalkyl, wie Methyl, Ethyl oder tert-Butyl, Niederalkylthioniederalkyl, wie tert-Butylthiomethyl, Niederalkylsulfinylniederalkyl, wie tert-Butylsulfinylmethyl, Niederalkylsulfonylniederalkyl, wie tert-Butylsulfonylmethyl, Phenylniederalkyl, wie Benzyl, Hydroxy, Niederalkoxy, wie Methoxy, Phenylniederalkoxy, wie Benzyloxy, Aminoniederalkyl, wie Aminomethyl oder 2-Amino-2-propyl, Niederalkylaminoniederalkyl, wie Methylaminomethyl, Niederalkanoylaminoniederalkyl, wie Acetylaminomethyl, Niederalkanoyl, wie Acetyl, Niederalkanoylniederalkyl, wie Acetylmethyl, Isobutyrylmethyl oder Pivaloylmethyl, oder Niederalkylcarbamoyl, wie Methylcarbamoyl, substituiert sein können, 2-Naphthylcarbonyl oder hydrierte Formen davon, wie z.B. 2-Decahydronaphthylcarbonyl, 2-(3-Indolyl)-acetyl oder 2-(3-Benzofuranyl)-acetyl, 3-Chroman- oder 3-Thiochromancarbonyl, oder Dibenzyloxyphosphoryl.

Ein Rest $R^1$, der als Analogon zum über Stickstoff gebundenen Rest der Aminosäure Tyrosin gelten kann, weist das Strukturelement der Formel

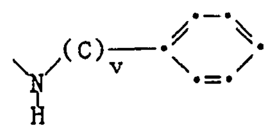
(Ic)

auf, worin der carbocyclische Ring ein Heteroatom, wie Stickstoff, enthalten kann und auch ganz oder teilweise gesättigt und/oder substituiert, beispielsweise durch Hydroxy oder durch Phenyl, sein kann, der Index v für null bis fünf, beispielsweise null bis drei, steht, eines der Kohlenstoffatome des carbocyclischen Ringes mit einem der Atome $-(C)_v-$ zu einem vorzugsweise fünf- oder sechsgliedrigen Ring verbunden sein kann und freie Valenzen Wasserstoff oder Substituenten tragen, beispielsweise Niederalkyl oder Hydroxyniederalkyl. Ferner kann ein solcher Rest $R^1$, der als Analogon zum Rest der Aminosäure Tyrosin gelten kann, auch Niederalkylamino sein.

Derartige Analoga $R^1$ sind beispielsweise Butylamino, Cyclohexylamino, 2-Decahydronaphthylamino, 6-Hydroxy-2-tetrahydronaphthylamino oder 2-Tetrahydronaphthylamino, 6-Hydroxy-2-naphthylamino oder 2-Naphthylamino, Pyridylmethylamino, Phenyl- oder Hydroxyphenylniederalkylamino, wie 2-Phenyl- oder Hydroxyphenylethylamino oder 5-Phenyl- oder Hydroxyphenylpentylamino, 1-Hydroxymethyl-2-hydroxyphenylethylamino oder Biphenylylniederalkylamino, wie 4-Biphenylylmethylamino.

Gleichermassen bevorzugte Verbindungen der Formel I sind dadurch gekennzeichnet, dass Q einen Rest der Teilformeln

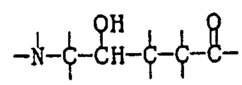
(IId)

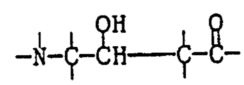
(IIe)

oder 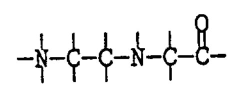
(IIm)

darstellt, worin freie Valenzen, die nicht an AAN bzw. AAC gebunden sind, unabhängig voneinander durch Wasserstoff, Niederalkyl, Arylniederalkyl oder Cycloalkylniederalkyl abgesättigt und/oder freie Valenzen an benachbarten N- und C-Atomen durch Niederalkylen überbrückt sind.

Besonders bevorzugte Verbindungen der Formel I sind dadurch gekennzeichnet, dass Q einen Rest der Teilformeln IId, IIe und IIm darstellt und freie Valenzen mit solchen Resten abgesättigt sind, dass die resultierenden Isostere den Dipeptidresten Leucin-Valin, Phenylalanin-Valin, Cyclohexylalanin-Valin, Leucin-Glycin, Cyclohexylalanin-Glycin, Tyrosin-Prolin, Phenylalanin-Prolin oder Cyclohexylalanin-Prolin entsprechen.

Die Verbindungen, deren Verwendung als gag-Protease-Hemmer hier offenbart ist, sind bekannt oder auf an sich bekannte Weise herstellbar, z.B. wie dies in den bereits angeführten Europäischen Patentanmeldungen beschrieben ist.

Beispielsweise werden sie hergestellt, indem man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppen mit Ausnahme der an der Reaktion teilnehmenden Gruppen gegebenenfalls in geschützter Form

vorliegen, unter Bildung einer Amidbindung kondensiert, und in einer erhältlichen Verbindung vorhandene Schutzgruppen abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Die vorliegende Erfindung betrifft auch die Verwendung von Verbindungen vom Typus von Reninhemmern, z.B. solchen der Formel I, zur Herstellung von pharmazeutischen Präparaten zur Anwendung als gag-Protease-Hemmer, beispielsweise zur Bekämpfung von durch Retroviren verursachten Krankheiten, z.B. von AIDS. So können solche Verbindungen zur Herstellung von pharmazeutischen Präparaten verwendet werden, welche eine wirksame Menge davon zusammen oder im Gemisch mit einer signifikanten Menge von anorganischen oder organischen, festen oder flüssigen, pharmazeutisch verwendbaren Trägerstoffen enthalten. Die Erfindung betrifft ferner auch die genannten pharmazeutischen Präparate zur Anwendung als gag-Protease-Hemmer beziehungsweise zur Bekämpfung von durch Retroviren verursachten Krankheiten, z.B. AIDS, selbst.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosie rung des Wirkstoffs hängt von der Warmblüter-Spezies, dem Körpergewicht, Alter und dem individuellen Zustand, der zu behandelnden Krankheit sowie von der Applikationsweise ab.

Die Erfindung betrifft auch eine Methode zur Behandlung von durch Retroviren verursachten Krankheiten, z.B. von AIDS, dadurch gekennzeichnet, dass eine therapeutisch wirksame Menge von Verbindungen vom Typus von Reninhemmern, z.B. solchen der Formel I, verabreicht wird. Die an Warmblüter, z.B. Menschen von etwa 70 kg Körpergewicht, zu verabreichenden Dosismengen liegen zwischen etwa 3 mg und etwa 3 g, vorzugsweise zwischen etwa 10 mg und etwa 1,5 g, z.B. bei ungefähr 300 mg bis 1000 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffs, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8 - 22, besonders 12 - 22, Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein- oder mehrwertiger, z.B. ein-, zwei-oder dreiwertiger Alkohol, z.B. Methanol, Ethanol, Propanol, Butanol oder Pentanol oder deren Isomere, vor allem aber Glycol oder Glycerin. Als Fettsäureester sind daher beispielsweise zu nennen: Ethyloleat, Isopropylmyristat, Isopropylpalmitat, "Labrafil M 2735" (Polyoxyethylenglycerintrioleat der Firma Gattefossé, Paris), "Myglyol 812" (Triglycerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff

mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und oder Polyethylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Ethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die folgenden Beispiele dienen zur Illustration der Erfindung, schränken deren Umfang jedoch in keiner Weise ein.

Temperaturen werden in Celsiusgraden angegeben. Die $R_f$-Werte werden auf Kieselgeldünnschichtplatten in folgenden Lösungsmittelsystemen ermittelt:

| | |
|---|---|
| A Essigester-n-Hexan | 1:1 |
| B Essigester-n-Hexan | 1:2 |
| C Essigester-n-Hexan | 1:4 |
| D Essigester-n-Hexan | 1:6 |
| E Essigester-n-Hexan | 1:9 |
| F Methylenchlorid-Methanol | 19:1 |
| G Methylenchlorid-Methanol | 9:1 |
| H Methylenchlorid-Methanol | 4:1 |
| I Methylenchlorid-Methanol-Wasser | 300:10:1 |
| J Methylenchlorid-Ether | 4:1 |
| K Methylenchlorid-Methanol-Ammoniak konz. | 40:10:1 |
| L Methylenchlorid-Methanol-Ammoniak konz. | 350:50:1 |
| M Methylenchlorid-Methanol-Wasser-Eisessig | 150:54:10:1 |
| N Methylenchlorid-Methanol-Wasser | 14: 6:1 |
| O Chloroform-Methanol-Wasser-Eisessig | 150:54:10:1 |
| P Chloroform-Methanol-Wasser-Eisessig | 180:20:2:1 |
| Q Chloroform-Methanol-Wasser-Eisessig | 170:26:3:1 |
| R Essigester | |
| S Methylenchlorid-Methanol-Ammoniak konz. | 800:50:1 |
| T Methylenchlorid-Methanol-Ammoniak konz. | 90:10:1 |
| U Toluol-Essigester | 4:1 |
| V Essigester-Methanol | 1:1 |
| W Essigester-n-Hexan | 5:2 |

Beispielsweise bedeutet die Abkürzung "$R_f$(A)" dass der $R_f$-Wert im System A ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Die Retentionszeiten ($t_{Ret}$) in der Hochdruckflüssig-Chromatographie (HPLC) werden auf einer 250 x 4,6 mm reversed phase $C_{18}$ Nucleosil® 5 µ Säule bei einer Fliessgeschwindigkeit von 1 ml/Min. ermittelt. Gradient A: 0 % → 100 % Acetonitril in Wasser enthaltend 0,05 % Trifluoressigsäure in 60 Min.; Gradient B: 10 % → 100 % Acetonitril in Wasser enthaltend 0,05 % Trifluoressigsäure in 40 Min.; Gradient C: 0 % → 90

% Acetonitril in Wasser enthaltend 1 % Trifluoressigsäure in 30 Min. Beispielsweise bedeutet die Abkürzung "$t_{Ret}$(A)", dass die Retentionszeit mit Gradient A ermittelt wurde.

Die Werte für Protonen-Kernresonanzspektroskopie ($^1$H-NMR) werden in ppm (parts per million) bezogen auf Tetramethylsilan als internen Standard angegeben. s = Singulett, d = Dublett, t = Triplett, q = Quartett, m = Multiplett, dd = Doppeldublett, br = breit. Die Werte für Infrarotspektren (IR) sind Wellenzahlen in $cm^{-1}$. Bei der "fast atom bombardment" Massenspektrometrie (FAB-MS) sind die Werte für die protonierte Masse $(M+H)^+$ angegeben.

Der Rest mit der Bezeichnung -Cha $\stackrel{c}{-}$ Val- bedeutet das zweiwertige Radikal von (2S,4S,5S)-5-Amino-6-cyclohexyl-4-hydroxy-2-isopropyl-hexansäure und hat die Formel

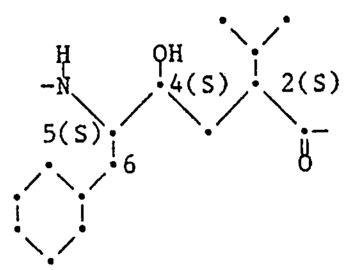

Der Rest mit der Bezeichnung -Cha $\stackrel{cx}{—}$ Val- leitet sich vom Rest -Cha $\stackrel{c}{-}$ Val-durch Ueberbrückung von NH und OH durch eine Isopropyliden-Gruppe ab und hat die Formel

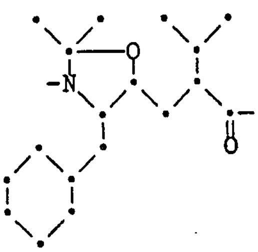

Der Rest mit der Bezeichnung -Leu $\stackrel{c}{-}$ Val- bedeutet entsprechend das zweiwertige Radikal von (2S,4S,5S)-5-Amino-4-hydroxy-2-isopropyl-7-methyl-octansäure. Sta ist das zweiwertige Radikal von (3S,4S)-4-Amino-3-hydroxy-6-methyl-heptansäure, Statin. ACHPA bedeutet das zweiwertige Radikal von (3S,4S)-4-Amino-5-cyclohexyl-3-hydroxy-pentansäure.

Der Rest mit der Bezeichnung -Phe $\stackrel{red}{—}$ Pro- bedeutet das zweiwertige Radikal von N-(2(S)-Amino-3-phenyl-propyl)-L-prolin und hat die Formel

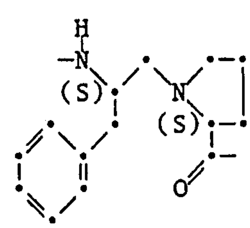

Entsprechend bedeutet -Cha $\stackrel{red}{—}$ Pro- das zweiwertige Radikal von N-(2(S)-Amino-3-cyclohexyl-propyl)-L-prolin und -Cha $\stackrel{red}{—}$ Val- das zweiwertige Radikal von N-(2(S)Amino-3-cyclohexyl-propyl)-L-valin.

Zur Bezeichnung von zweiwertigen Radikalen von natürlichen α-Aminosäuren werden die in der Peptidchemie üblichen Abkürzungen verwendet. An der phenolischen Hydroxygruppe mit dem Rest R veretherte Tyrosin-Radikale werden mit Tyr(OR) bezeichnet. Nle bedeutet das Radikal von Norleucin, Cha das Radikal von Cyclohexylalanin.

Weitere Abkürzungen:

abs. = absolut (wasserfrei)
BBSP = 2(S)-Benzyl-3-tert-butylsulfonyl-propionyl
BOC = tert-Butoxycarbonyl
BOP = Benzotriazol-1-yloxytris(dimethylamino)phosphonium-hexafluorophosphat
DMA = Dimethylacetamid
DCCI = Dicyclohexylcarbodiimid
DCH = Dicyclohexylharnstoff
DMF = Dimethylformamid
DMSO = Dimethylsulfoxid
Fmoc = 9-Fluorenylmethoxycarbonyl
ges. = gesättigt
HBTU = O-Benzotriazol-1-yl-N,N,N′,N′-tetramethyluronium-hexafluorophosphat
HOBH = N-Hydroxy-endo-norbornan-2,3-dicarbonsäureimid
HOBt = 1-Hydroxybenzotriazol
konz. = konzentriert
Min. = Minute(n)
Sdp. = Siedepunkt
Smp. = Schmelzpunkt
Std. = Stunde(n)
Tcp = Trichlorphenyl
THF = Tetrahydrofuran
Z = Benzyloxycarbonyl

Beispiel 1: Z-Arg-Arg-Pro-Phe-Val-Cha $\overset{c}{-}$ Val-Val-Tyr-OMe

Eine Mischung aus 160 mg H-Phe-Val-Cha $\overset{c}{-}$ Val-Val-Tyr-OMe, 180 mg Z-Arg-Arg-Pro-OH•HCl, 42 mg Triethylaminhydrochlorid, 62 mg DCCI, 42 mg HOBt und 10 ml DMF wird 48 Std. bei Raumtemperatur gerührt. Der kristallisierte DCH wird abfiltriert und das Filtrat eingedampft. Der Rückstand wird in Methanol gelöst und mit Diisopropylether gefällt. Das Rohprodukt wird anschliessend durch Flash-Chromatographie an 20 g Kieselgel (Laufmittel M) gereinigt. $R_f(M)$ = 0,25; FAB-MS: $(M+H)^+$ = 1337; $t_{Ret}(A)$ = 35,2 Min.

Die Ausgangsmaterialien werden folgendermassen hergestellt:

a) 2(S)-Benzyloxycarbonylamino-3-cyclohexyl-propionsäureethylester:

243 g 2(S)-Benzyloxycarbonylamino-3-cyclohexyl-propionsäure (Herstellung: Helvetica Chimica Acta 57, 2131(1974)) werden in 600 ml Toluol und 900 ml Ethanol vorgelegt. Das Reaktionsgemisch wird auf 0° gekühlt und 88,3 g Thionylchlorid innerhalb 30 Min. zugetropft. Die Kühlung wird entfernt und die Mischung während 18 Std. gerührt. Das Reaktionsgemisch wird filtriert und das Filtrat eingeengt. Der Rückstand wird mittels Flash-Chromatographie (2 kg Kieselgel 60, 40-63 µm, Laufmittel E) aufgetrennt. Die produkthaltigen Fraktionen werden vereinigt, eingedampft und im Hochvakuum getrocknet. Man erhält die Titelverbindung als leicht gelbliches Oel. $R_f$ (E) = 0,2; $R_f$ (B) = 0,52.

b) 2(S)-Benzyloxycarbonylamino-3-cyclohexyl-propanal:

116,1 g 2(S)-Benzyloxycarbonylamino-3-cyclohexyl-propionsäureethylester werden in 2,2 1 Toluol vorgelegt und auf -65° abgekühlt. 836 ml Diisobutylaluminiumhydrid werden innerhalb 30 Min. tropfenweise bei -65° zugegeben und das Gemisch 20 Min. nachgerührt. Dann werden bei -65° 84,2 ml Methanol innerhalb 10 Min. zugetropft, anschliessend 825 ml wässrige Kalium-natrium-tartrat-Lösung ohne Kühlung. Das Reaktionsgemisch wird auf 3 l Kalium-natrium-tartrat-Lösung/Eis ausgetragen und mit 5 l Ether extrahiert. Die Etherphase wird mit 2 l Wasser gewaschen, dann sofort in eine Lösung bestehend aus 106 g Semicarbazid-Hydrochlorid und 156,5 g Natriumacetat in 620 ml Wasser und 620 ml Ethanol gegossen. Das Reaktionsgemisch wird 1 Std. bei Raumtemperatur nachgerührt, dann im Scheidetrichter abgetrennt und die Wasserphase mit 2 x 1,5 l Ether extrahiert. Die organische Phase wird über Magnesiumsulfat getrocknet und eingedampft. Das Rohprodukt wird mittels Flash-Chromatographie gereinigt (2 kg Kieselgel

60, 40-63 µm, Laufmittel A). Durch Eindampfen der vereinigten, produkthaltigen Fraktionen erhält man das Semicarbazon der Titelverbindung, $R_f$ (G) = 0,51. 130 g dieses Semicarbazons werden in 1 l THF gelöst, mit 282 ml 37 % Formaldehydlösung und dann bei 10° mit 143 ml 0,5N HCl versetzt. Das Reaktionsgemisch wird 2 Std. bei Raumtemperatur gerührt, filtriert und das Filtrat mit 0,5 l Wasser, 0,5 l $NaHCO_3$ und 0,5 l Wasser gewaschen. Die Wasserphasen werden mit 600 ml Ether extrahiert. Die Etherphasen werden über Magnesiumsulfat getrocknet und eingedampft. Der Rückstand wird mit 100 ml Toluol versetzt und eingedampft, wobei die Titelverbindung erhalten wird. Diese wird sofort weiterverarbeitet.

c) (1(S)-Benzyloxycarbonylamino-2-cyclohexyl-ethyl)-oxiran:

18,9 g Natriumhydriddispersion (55 % in Oel) werden in einem trockenen Sulfierkolben unter Argon durch dreimaliges Aufrühren in 50 ml Petrolether (Sdp. 40-60°) und anschliessendes Abdekantieren des Lösungsmittels vom Oel befreit. Nach Trocknen im Hochvakuum wird ein graues Pulver erhalten, das in 500 ml THF vorgelegt und mit 55,6 g Trimethylsulfoxoniumiodid versetzt wird, wobei die Temperatur auf ca. 40° steigt. Die graue Suspension wird am Rückfluss 1 Std. gekocht und anschliessend innerhalb von 50 Min. bei -70° mit einer Lösung von 108,6 g 2(S)-Benzyloxycarbonylamino-3-cyclohexyl-propanal in 250 ml THF versetzt. Die gelbe Suspension wird 2 Std. bei 0° gerührt. Die gelblich-trübe Lösung wird auf 500 g Eis gegossen. Die wässrige Lösung wird mit 2,5 l Ether extrahiert, die organische Phase mit Wasser gewaschen und nach Trocknen über Natriumsulfat eingedampft. Der ölige Rückstand wird mittels Flash-Chromatographie (2,5 kg Kieselgel 60, 40-63 µm, Laufmittel C) aufgetrennt. Die produkthaltigen Fraktionen werden vereinigt, eingedampft und im Hochvakuum getrocknet. Man erhält die Titelverbindung (Diastereomerengemisch, ca. 4:1) als leicht gelbliches Oel. $R_f$(I) = 0,71; $R_f$(C) = 0,16.

d) 3(S)-Benzyloxycarbonylamino-4-cyclohexyl-1-iod-butan-2(R,S)-ol:

42,3 g (1(S)-Benzyloxycarbonylamino-2-cyclohexyl-ethyl)-oxiran werden in 200 ml Acetonitril aufgenommen und die erhaltene Lösung auf 0° abgekühlt. Nach Zugabe von 20,9 g Natriumiodid werden während 30 Min. tropfenweise bei 0° 17,7 ml Trimethylchlorsilan zugegeben. Das Gemisch wird 40 Min. bei 0 - 3° gerührt und anschliessend auf 700 ml eiskaltes Wasser gegossen. Das wässrige Gemisch wird mit Ether extrahiert und die organische Phase mit 750 ml 5 %-iger wässriger Natriumthiosulfatlösung und 750 ml gesättigter, wässriger Natriumchloridlösung gewaschen. Nach Trocknen über Natriumsulfat und Eindampfen erhält man ein öliges Gemisch der Titelverbindung, die direkt weiterverarbeitet wird.

e) 3-Benzyloxycarbonyl-4(S)-cyclohexylmethyl-2,2-dimethyl-5(R)-iod-methyl-1,3-oxazolidin:

49,3 g der Verbindung Beispiel 1d) und 1,07 g p-Toluolsulfonsäuremonohydrat werden in 140 ml 2,2-Dimethoxypropan und 450 ml Methylenchlorid 3 Std. lang bei Raumtemperatur gerührt. Das Gemisch wird zwischen 1 l Methylenchlorid und 500 ml gesättigter, wässriger Natriumhydrogencarbonatlösung ausgeschüttelt. Die organische Phase wird mit Wasser gewaschen, über Natriumsulfat getrocknet und eingedampft. Das Rohprodukt wird mittels Flash-Chromatographie gereinigt (3 kg Kieselgel 60, 40-63 µm, Laufmittel D). Durch Eindampfen der vereinigten, produkthaltigen Fraktionen erhält man die Titelverbindung als leicht gelbliches Oel. $R_f$ (C) = 0,55; $R_f$ (D) = 0,46.

f) 2(R,S)-(3-Benzyloxycarbonyl-4(S)-cyclohexylmethyl-2,2-dimethyl-l,3-oxazolidinyl-5-(S)-methyl)-3-methylbuttersäuremethylester:

14,3 ml Diisopropylamin werden in 200 ml absolutem Tetrahydrofuran unter Argon gelöst und auf 0° abgekühlt. Anschliessend wird bei 0 - 5° das Gemisch 20 Min. lang tropfenweise mit 65,8 ml einer 1,6M Lösung von n-Butyllithium in Hexan versetzt und 20 Min. gerührt. Dann werden bei -70° bis -75° 13,3 ml Isovaleriansäuremethylester zugetropft und die Mischung 1,5 Std. bei -75° gerührt. Bei -60° bis -75° werden unter Rühren 320 ml Hexamethylphosphorsäuretriamid zugetropft. Die entstandene Suspension wird 10 Min. lang gerührt und schliesslich bei -70° bis -75° in 5 Min. tropfenweise mit einer Lösung von 43,4 g der Verbindung Beispiel 1e) in 110 ml Tetrahydrofuran versetzt. Das Reaktionsgemisch wird bei Raumtemperatur während 2,5 Std. gerührt und schliesslich auf ein Gemisch von 1 l gesättigter, wässriger Ammoni-

umchloridlösung und 500 g Eis gegossen. Die wässrige Phase wird mit 2 l Essigester extrahiert, die organische Phase mit Wasser gewaschen und über Natriumsulfat getrocknet. Nach Eindampfen erhält man das Diastereomerengemisch der Titelverbindung als gelbes Oel. $R_f$ (C) = 0,36; $R_f$ (E) = 0,21 (Werte für die weniger polare Komponente).

g) 2(R,S)-(3-Benzyloxycarbonyl-4(S)-cyclohexylmethyl-2,2-dimethyl-1,3-oxazolidinyl-5(S)-methyl)-3-methylbuttersäure:

16,5 g Kalium-tert-butylat werden in 250 ml Ether bei ca. 5° mit 1,77 ml Wasser versetzt. Die weisse Suspension wird noch 10 Min. im Eisbad gerührt und darauf mit 35,8 g der Verbindung Beispiel 1f) (Diastereomerengemisch) in 250 ml Ether versetzt, wobei die Temperatur unterhalb 10° gehalten wird. Das Reaktionsgemisch wird nun während 18 Std. bei Raumtemperatur gerührt und schliesslich auf 500 ml gesättigte, wässrige Ammoniumchloridlösung gegossen. Die wässrige Phase wird mit Essigester extrahiert und die organische Phase mit gesättigter, wässriger Natriumchloridlösung gewaschen, über Natriumsulfat getrocknet und eingedampft. Das ölige Rohprodukt wird durch Flash-Chromatographie aufgetrennt (2,5 kg Kieselgel 60, 40 - 63 µm, Laufmittel C). Z-Cha $\overset{cx}{—}$ Val-OH, die weniger polare Komponente der Titelverbindung mit der gewünschten Konfiguration des an die Isopropylgruppe gebundenen C-Atoms (S-Konfiguration) wird als gelbes Oel erhalten. $R_f$ (I) = 0,20; $R_f$ (J) = 0,35.

h) HCl•H-Val-Tyr-OMe:

10,0 g Z-Val-Tyr-OMe werden in 200 ml Methanol und 24 ml 1 N HCl in Gegenwart von 1,0 g Palladium-Kohle (10 % Pd) bei Normaldruck und Raumtemperatur bis zur Sättigung hydriert. Das Reaktionsgemisch wird filtriert, und das Filtrat wird eingedampft und getrocknet. $R_f$ (N) = 0,64.

i) Z-Cha $\overset{cx}{—}$ Val-Val-Tyr-OMe:

Eine Mischung aus 2,06 g HCl•H-Val-Tyr-OMe, 3,12 g Z-Cha $\overset{cx}{—}$ Val-OH, 1,74 g DCCI, 1,22 g HOBt, 0,75 g N-Methylmorpholin und 50 ml DMF wird 16 Std. bei Raumtemperatur gerührt. Der DCH wird abfiltriert, das Filtrat eingeengt und am Hochvakuum getrocknet. Der Rückstand wird mittels Flash-Chromatographie (100 g Kieselgel 60, Laufmittel B) gereinigt. $R_f$ (A) = 0,36.

j) H-Cha $\overset{c}{—}$ Val-Val-Tyr-OMe:

3,6 g Z-Cha $\overset{cx}{—}$ Val-Val-Tyr-OMe werden in 80 m Methanol-Wasser 9:1 in Gegenwart von 360 mg Palladium-Kohle (10 % Pd) bei Normaldruck und Raumtemperatur bis zur Sättigung hydriert. Das Reaktionsgemisch wird filtriert, und das Filtrat wird mit 30 ml Wasser verdunnt und für 5 Std. bei Raumtemperatur gerührt. Nach Abdampfen des Lösungsmittels wird die Titelverbindung aus Dioxan/Wasser 9:1 lyophilisiert. $R_f$ (H) = 0,10.

k) Boc-Val-Cha $\overset{c}{—}$ Val-Val-Tyr-OMe

wird ausgehend von 1,45 g H-Cha $\overset{c}{—}$ Val-Val-Tyr-OMe, 630 mg BOC-Val-OH, 530 mg HOBH und 660 mg DCCI analog Beispiel 1i) erhalten und durch Umfällung aus Methanol-$NaHCO_3$-Lösung gereinigt. $R_f$ (G) = 0,75.

l) HCl•H-Val-Cha $\overset{c}{—}$ Val-Val-Tyr-OMe:

2,16 g BOC-Val-Cha $\overset{c}{—}$ Val-Val-Tyr-Ome werden in 20 ml 4,3N HCl in Dioxan gelöst und für 30 Min. bei Raumtemperatur gerührt. Das Reaktionsgemisch wird eingedampft, und der Rückstand wird durch Flash-Chromatographie (60 g Kieselgel 60, Laufmittel G) gereinigt. $R_f$ (G) = 0,18.

m) Fmoc-Phe-Val-Cha $\overset{c}{-}$ Val-Val-Tyr-OMe:

Eine Mischung aus 240 mg HCl•H-Val-Cha $\overset{c}{-}$ Val-Val-Tyr-OMe, 170 mg Fmoc-Phe-OTcp, 0,15 ml N-Ethyldiisopropylamin und 10 ml DMF wird 20 Std. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert.

n) H-Phe-Val-Cha $\overset{c}{-}$ Val-Val-Tyr-Ome:

Eine Lösung aus 305 mg Fmoc-Phe-Val-Cha $\overset{c}{-}$ Val-Val-Tyr-OMe, 5 ml Piperidin und 5 ml DMF wird für 60 Min. bei Raumtemperatur gerührt und danach eingedampft. Der Rückstand wird in wenig Methylenchlorid gelöst und mit Diisopropylether gefällt. Der Niederschlag wird bei 0° abfiltriert und anschliessend aus Dioxan/Wasser 9:1 lyophilisiert. $R_f$ (M) = 0,7.

Beispiel 2: BBSP-Val-Cha $\overset{c}{-}$ Val-Val-Tyr-OMe

Analog Beispiel 1 wird die Titelverbindung ausgehend von 760 mg HCl•H-Val-Cha $\overset{c}{-}$ Val-Val-Tyr-OMe (Beispiel 1 l), 350 mg 2(S)-Benzyl-3-tert-butylsulfonylpropionsäure (BBSP-OH), 190 mg HOBt und 280 mg DCCI hergestellt und durch Flash-Chromatographie gereinigt. $R_f$ (G) = 0,50; FAB-MS: $(M+H)^+$ = 913; $t_{Ret}$-(A) = 42,1 Min.

Das Ausgangsmaterial wird wie folgt hergestellt:

a) 2(S)-Benzyl-3-tert-butylsulfonyl-propionsäure (BBSP-OH):

142 g 2(R,S)Benzyl-3-tert-butylsulfonyl-propionsäure werden mit 85,7 g (+)-Dehydroabietylamin und 27,8 ml Triethylamin in 2 l Isopropanol umgesetzt. Nach viermaliger Kristallisation des Salzes aus heissem Isopropanol wird die Säure wieder durch Extraktion mit verdünnter Natriumcarbonatlösung und anschliessendem Ansäuren mit verdünnter Salzsäure freigesetzt. Durch Umkristallisation aus Essigester/Hexan wird die Titelverbindung in hoher optischer Reinheit erhalten (über 98 % ee): $[\alpha]_D^{20}$ = -10,9° (c = 0,91 in $CH_2Cl_2$); Smp. 99-101°; $R_f$ (A) = 0,16; $R_f$ (B) = 0,4.

b) 2(R,S)-Benzyl-3-tert-butylsulfonyl-propionsäure:

91,9 g 2-Benzyl-3-tert-butylsulfonyl-propionsäureethylester werden in 500 ml 6 N Salzsäure und 100 ml Essigsäure 15 Std. am Rückfluss gekocht. Beim Abkühlen kristallisiert die Titelverbindung direkt aus dem Reaktionsgemisch. Smp. 147-8°; $R_f$ (B) = 0,4; $^1$H-NMR ($CDCl_3$): 1,35 (s, 9H); 2,97 (m, 1H); 3,05 (dd, 1H); 3,22 (dd, 1H); 3,45 (m, 2H); 7,25 (m, 5H); 8,5 (br s, 1H).

c) 2-Benzyl-3-tert-butylsulfonyl-propionsäure-ethylester:

60 g α-Benzylacrylsäure-ethylester werden in 600 ml Ethanol gelöst und mit 0,83 g Natriummethylat und 37 ml tert-Butylmercaptan umgesetzt. Die Mischung wird 24 Std. bei Raumtemperatur gerührt, mit 500 ml 0,04 N wässeriger Schwefelsäure verdünnt und unter Eiskühlung mit 260 g Oxone® (Kaliumper oxomonosulfat, 50 % $KHSO_5$, Ventron) versetzt. Das Reaktionsgemisch wird über Nacht bei Raumtemperatur gerührt, dann filtriert und eingeengt. Die wässrige Lösung wird mit Methylenchlorid extrahiert, die Extrakte über Natriumsulfat getrocknet und eingedampft. Smp. 47-48°; $^1$H-NMR ($CDCl_3$): 1,13 ppm (t, 3H); 1,38 (s, 9H); 2,95 (dd, 1H); 3,01 (dd, 1H); 3,10 (dd, 1H); 3,42 (dd, 1H); 3,46 (dd, 1H); 4,12 (q, 2H); 7,25 (m, 5H).

d) α-Benzylacrylsäure-ethylester:

20 g Benzylmalonsäurediethylester in 40 ml Ethanol werden bei Raumtemperatur mit 4,0 g KOH in 50

ml Ethanol versetzt, über Nacht bei Raumtemperatur gerührt, eingedampft, mit 7,1 ml Wasser versetzt und im Eisbad mit 6,3 ml konz. Salzsäure angesäuert. Es wird zwischen Wasser und Ether verteilt, die organische Phase getrocknet und der Ether abdestilliert. Der Rückstand wird mit 12,9 ml Pyridin, 0,61 g Piperidin und 1,78 g Paraformaldehyd versetzt. Das Gemisch wird im Oelbad (130°) während 90 Min. erhitzt, abgekühlt, mit 220 ml Wasser versetzt und dreimal mit 75 ml n-Hexan extrahiert. Die vereinigten organischen Phasen werden mit Wasser, 1 N HCl, Wasser, ges. $NaHCO_3$-Lösung und Sole gewaschen. Die Titelverbindung wird durch Destillation gewonnen. $^1H$-NMR (DMSO-$d_6$): 1,2 ppm (t, 3H); 3,6 (d, 2H); 4,1 (q, 2H); 5,6 (m, 1H); 6,15 (m, 1H); 7,25 (m, 5H).

Beispiel 3: BBSP-Val-Cha $\overset{c}{-}$ Val-Val-Tyr-OH

Eine Mischung aus 65 mg BBSP-Cha $\overset{c}{-}$ Val-Val-Tyr-OMe (Beispiel 2), 2 ml 2 N NaOH und 3 ml Dioxan wird 20 Min. bei Raumtemperatur gerührt. Das Reaktionsgemisch wird anschliessend mit 2 ml 2 N HCl verdünnt und lyophilisiert. Der Rückstand wird mittels Flash-Chromatographie (8 g Kieselgel, Laufmittel H) gereinigt. $R_f$ (N) = 0,53.

Beispiel 4: BBSP-Val-Cha $\overset{c}{-}$ Val-Val-Tyr-$NH_2$

Analog Beispiel 2 wird die Titelverbindung ausgehend von 30 mg HCl•H-Val-Cha $\overset{c}{-}$ Val-Val-Tyr-$NH_2$, 15 mg 2(S)-Benzyl-3-tert-butylsulfonylpropionsäure (BBSP-OH), 8 mg HOBt und 12 mg DCCl hergestellt und durch Flash-Chromatographie an 7 g Kieselgel 60 (Laufmittel F) gereinigt. $R_f$ (G) = 0,32; FAB-MS: $(M+H)^+$ = 898; $t_{Ret}(A)$ = 41,7 Min.

Die Ausgangsmaterialien werden folgendermassen hergestellt:

a) HCl•H-Val-Cha $\overset{c}{-}$ Val-Val-Tyr-$NH_2$

wird ausgehend von 360 mg BOC-Val-Cha $\overset{c}{-}$ Val-Val-Tyr-$NH_2$ und 5,0 ml 4,3 N HCl in Dioxan analog Beispiel 1 l) hergestellt. $R_f$ (L) = 0,3.

b) BOC-Val-Cha $\overset{c}{-}$ Val-Val-Tyr-$NH_2$

wird ausgehend von 300 mg H-Cha $\overset{c}{-}$ Val-Val-Tyr-$NH_2$, 147 mg BOC-Val-OH, 122 mg HOBH und 268 mg DCCl analog Beispiel 1 k) erhalten. $R_f$ (G) = 0,33.

c) H-Cha $\overset{c}{-}$ Val-Val-Tyr-$NH_2$

wird durch Hydrierung von 600 mg Z-Cha $\overset{cx}{-}$ Val-Val-Tyr-$NH_2$ in Gegenwart von 60 mg Palladium-Kohle (10 % Pd) analog Beispiel 1 j) erhalten. $R_f$ (L) = 0,06.

d) Z-Cha $\overset{cx}{-}$ Val-Val-Tyr-$NH_2$:

Eine Mischung aus 772 mg Z-Cha $\overset{cx}{-}$ Val-Val-Tyr-OH, 590 mg $NH_4Cl$, 315 mg DCCl, 217 mg HOBt und 10 ml DMF wird mit $NaHCO_3$ auf pH 5,5-6,0 gestellt und anschliessend für 16 Std. gerührt. Der DCH wird abfiltriert und das Filtrat eingedampft. Der Rückstand wird durch Flash-Chromatographie an 40 g Kieselgel 60 (Laufmittel F) gereinigt. $R_f$ (G) = 0,40.

e) Z-Cha $\overset{cx}{-}$ Val-Val-Tyr-OH

wird ausgehend von 790 mg Z-Cha $\overset{cx}{-}$ Val-Val-Tyr-OMe (Beispiel 1 i), 4 ml 4 N NaOH und 16 ml Dioxan analog Beispiel 3 hergestellt. $R_f$ (G) = 0,07.

Beispiel 5: N-(3-Amino-3,3-dimethylpropanoyl)-Tyr(OMe)-Val-Cha $\overset{c}{-}$ Val-Val-Tyr-$NH_2$

Eine Lösung von 120 mg N-( 3-tert-Butoxycarbonylamino-3,3-dimethylpropanoyl)-Tyr(OMe)-Val-Cha $\overset{c}{-}$

Val-Val-Tyr-$NH_2$ in 1,0 ml 95%iger wässriger Trifluoressigsäure wird für 20 Min. gerührt. Das Reaktionsgemisch wird eingedampft, und der Rückstand wird durch Flash-Chromatographie an 10 g Kieselgel 60 (Laufmittel G) gereinigt. $R_f$ (L) = 0,06, $R_f$ (K) = 0,46; FAB-MS: $(M+H)^+$ = 908; $t_{Ret}(A)$ = 35,6 Min.

Die Ausgangsmaterialien werden folgendermassen hergestellt:

a) N-(3-tert-Butoxycarbonylamino-3,3-dimethylpropanoyl)-Tyr(OMe)-Val-Cha$\overset{c}{-}$Val-Val-Tyr-$NH_2$

wird ausgehend von 90 mg H-Tyr(OMe)-Val-Cha$\overset{c}{-}$Val-Val-Tyr-$NH_2$, 31 mg 3-tert-Butoxycarbonylamino-3,3-dimethylpropionsäure, 26 mg HOBt und 34 mg DCCI analog Beispiel 1 i) hergestellt und durch Flash-Chromatographie an 8 g Kieselgel 60 (Laufmittel G) gereinigt. $R_f$ (G) = 0,30.

b) H-Tyr(OMe)-Val-Cha$\overset{c}{-}$Val-Val-Tyr-$NH_2$

wird ausgehend von 260 mg HCl•H-Val-Cha$\overset{c}{-}$Val-Val-Tyr-$NH_2$ (Beispiel 4a), 320 mg Fmoc-Tyr(OMe)-OTcp und 0,15 ml N-Ethyldiisopropylamin analog Beispiel 1m) und 1n) hergestellt.

c) Fmoc-Tyr(OMe)-OTcp:

Zu einer Lösung von 1,45 g Fmoc-Tyr(OMe)-OH in 30 ml abs. THF werden bei 0° 0,76 g Trichlorphenol und 0,82 g DCCI gegeben, und das Reaktionsgemisch wird für 1/2 Std. bei 0° und anschliessend für 2 Std. bei Raumtemperatur gerührt. Der DCH wird bei 0° abfiltriert, das Filtrat wird eingedampft, und der Rückstand wird aus THF/Hexan kristallisiert.

Beispiel 6: Z-Arg-Arg-Pro-Tyr(OMe)-Val-Cha$\overset{c}{-}$Val-Val-Tyr-$NH_2$

Analog Beispiel 1 wird die Titelverbindung ausgehend von 150 mg H-Tyr(OMe)-Val-Cha$\overset{c}{-}$Val-Val-Tyr-$NH_2$, 150 mg Z-Arg-Arg-Pro-OH•HCl, 35 mg Triethylammoniumchlorid, 50 mg HOBt und 68 mg DCCI hergestellt. $R_f$ (M) = 0,24; $t_{Ret}(A)$ = 36,3 Min.

Beispiel 7: N-(2(R,S)-Benzyl-3-pivaloyl-propionyl)-Nle-Cha$\overset{c}{-}$Val-Val-Tyr-OMe

Eine Lösung von 20 mg HCl•H-Nle-Cha$\overset{c}{-}$Val-Val-Tyr-OMe, 22 mg (R,S)-2-Benzyl-3-pivaloylpropionsäure (J. Med. Chem. 31, 1839 (1988)), 33 mg HBTU und 24 µl Triethylamin in 2 ml DMF wird 2 Std. bei Raumtemperatur gerührt. Die Mischung wird vollständig eingedampft, der Rückstand mit ges. Natriumbicarbonat-Lösung und Diisopropylether gewaschen und schliesslich in DMF gelöst. Das Produkt wird mit Diisopropylether gefällt und ergibt nach Lyophilisation aus Dioxan/tert-Butanol/Wasser die Titelverbindung als Diastereomerengemisch; FAB-MS: $(M+H)^+$ = 891; $R_f$ (P) = 0,55; HPLC: 2 Diastereomere im Verhältnis 1,8:1, $t_{Ret}(B)$ = 30,0 und 30,4 Min.

Das Ausgangsmaterial wird folgendermassen hergestellt:

a) HCl•H-Nle-Cha$\overset{c}{-}$Val-Val-Tyr-OMe

wird ausgehend von 50 mg BOC-Nle-Cha$\overset{c}{-}$Val-Val-Tyr-OMe und 5 ml 4H HCl in Dioxan analog Beispiel 1l) hergestellt. Das Produkt wird aus Dioxan lyophilisiert. $R_f$ (P) = 0,15; $t_{Ret}(B)$ = 19,9 Min.

b) BOC-Nle-Cha$\overset{c}{-}$Val-Val-Tyr-OMe:

Eine Lösung von 60 mg H-Cha$\overset{c}{-}$Val-Val-Tyr-OMe (Beispiel 1j), 28 mg BOC-Nle-OH, 46 mg HBTU und 35 µl Triethylamin in 2 ml DMF wird 3 Std. bei Raumtemperatur gerührt. Die Mischung wird eingedampft, der Rückstand mit ges. Natriumbicarbonat-Lösung und Diisopropylether gewaschen und schliesslich in DMF gelöst. Das Produkt wird mit Diisopropylether gefällt und aus Dioxan/tert-Butanol/Wasser lyophilisiert. FAB-MS: $(M+H)^+$ = 761; $R_f$ (P) = 0,50; $t_{Ret}$ (B) = 27,9 Min.

Beispiel 8: BBSP-Nle-Cha$\overset{C}{-}$Val-Val-Tyr-OMe

Analog Beispiel 7 wird die Titelverbindung ausgehend von 20 mg HCl•H-Nle-Cha$\overset{C}{-}$Val-Val-Tyr-OMe (Beispiel 7a), 25 mg 2(S)-Benzyl-3-tert-butylsulfonylpropionsäure (BBSP-OH), 33 mg HBTU und 24 µl Triethylamin hergestellt, durch Flash-Chromatographie an 10 g Kieselgel (Laufmittel P) gereinigt und aus Dioxan/tert-Butanol/Wasser lyophilisiert. FAB-MS: $(M+H)^+$ = 927; $R_f$ (P) = 0,46; $t_{Ret}$(B) = 28,0 Min.

Beispiel 9: BBSP-Tyr-Cha$\overset{C}{-}$Val-Val-Tyr-OMe

Eine Lösung von 17 mg BBSP-Tyr(OtBu)-Cha$\overset{C}{-}$Val-Val-Tyr-OMe in 2 ml 95 %iger wässriger Trifluoressigsäure wird 2 Std. bei Raumtemperatur gerührt. Die Mischung wird eingedampft und der Rückstand durch Chromatographie an 10 g Kieselgel (Laufmittel P) gereinigt. Die produkthaltigen Fraktionen werden eingedampft, der Rückstand in DMF gelöst und mit Diisopropylether gefällt. Die Titelverbindung wird aus Dioxan/tert-Butanol/Wasser lyophilisiert. FAB-MS: $(M+H)^+$ = 977; $R_f$ (P) = 0,40; $t_{Ret}$(B) = 25,5 Min.

Das Ausgangsmaterial wird folgendermassen hergestellt:

a) BBSP-TYr(OtBu)-Cha$\overset{C}{-}$Val-Val-Tyr-OMe

wird analog Beispiel 7 aus 20 m-H-Tyr(OtBu)-Cha$\overset{C}{-}$Val-Val-Tyr-OMe, 22 mg 2(S)-Benzyl-3-tertbutylsulfonylpropionsäure (BBSP-OH), 30 mg HBTU und 22 µl Triethylamin hergestellt. Das Rohprodukt wird durch Flash-Chromatographie an 10 g Kieselgel (Laufmittel P) gereinigt und aus Dioxan/tert-Butanol/Wasser lyophilisiert. $R_f$ (P) = 0,55.

b) H-Tyr(OtBu)-Cha$\overset{C}{-}$Val-Val-Tyr-OMe:

Eine Lösung von 70 mg Fmoc-Tyr(OtBu)-Cha$\overset{C}{-}$Val-Val-Tyr-OMe in 2 ml Dimethylacetamid/Piperidin 30:20 (v/v) wird 30 Min. bei Raumtemperatur gerührt. Die Mischung wird eingedampft, der Rückstand in DMF gelöst und mit Diisopropylether gefällt. Der ölige Rückstand wird mit Methylenchlorid gewaschen, in DMF gelöst und bei 0° erneut mit Diisopropylether gefällt und ergibt die Titelverbindung als amorphen Festkörper. $R_f$ (P) = 0,30; $t_{Ret}$(B) = 22,3 Min.

c) Fmoc-Tyr(OtBu)-Cha$\overset{C}{-}$Val-Val-Tyr-OMe

wird analog Beispiel 7 ausgehend von 60 mg H-Cha$\overset{C}{-}$Val-Val-Tyr-OMe, 55 mg Fmoc-Tyr(OtBu)-OH, 46 mg HBTU und 35 µl Triethylamin hergestellt. Das Rohprodukt wird durch Flash-Chromatographie an 20 g Kieselgel (Laufmittel P) gereinigt. Die produkthaltigen Fraktionen werden eingedampft und der Rückstand aus DMF/Diisopropylether gefällt. $R_f$ (P) = 0,60; $t_{Ret}$(B) = 32,4 Min.

Beispiel 10: N-(2(R,S)-Benzyl-3-pivaloyl-propionyl)-Ala-Cha$\overset{C}{-}$Val-Val-Tyr-OMe

Eine Mischung aus 185 mg rohem H-Ala-Cha$\overset{C}{-}$Val-Val-Tyr-OMe, 17,5 mg 2(R,S)-Benzyl-3-pivaloylpropionsäure, 12,8 mg HOBH, 15,6 mg DCCI und 0,64 ml DMF wird 16 Std. bei Raumtemperatur gerührt. Analog Beispiel 1 wird aufgearbeitet, wobei nach Chromatographie (Kieselgel 60, Laufmittel Essigester) ein Diastereomerengemisch der Titelverbindung erhalten wird. FAB-MS: $(M+H)^+$ = 849,7; $R_f$ (R) = 0,7; $t_{Ret}$(B) = 27,5 und 27,9 Min.

Das Ausgangsmaterial wird folgendermassen hergestellt:

a) H-Ala-Cha$\overset{C}{-}$Val-Val-Tyr-OMe:

Eine Lösung von 90 mg BOC-Ala-Cha$\overset{C}{-}$Val-Val-Tyr-OMe in 2,3 ml 95 %iger wässriger Trifluoressigsäure wird 1,5 Std. bei 0° gerührt. Das Reaktionsgemisch wird im Eisbad mit wenig Wasser versetzt und die Trifluoressigsäure mit festem $NaHCO_3$ neutralisiert. Der Rückstand wird mit Essigester mehrmals gewaschen, die organischen Phasen vereinigt und über Natriumsulfat getrocknet. Nach dem Eindampfen wird die rohe Titelverbindung erhalten. $R_f$ (R) = 0.

b) BOC-Ala-Cha $\overset{C}{-}$ Val-Val-Tyr-OMe:

Eine Mischung aus 109,5 mg H-Cha $\overset{C}{-}$ Val-Val-Tyr-OMe (Beispiel 1j), 41,6 mg BOC-Ala-OH, 49,6 mg DCCI, 39,8 mg HOBH und 2 ml DMF wird 5 Std. bei Raumtemperatur gerührt. Nach Kühlen auf 0° und Filtration wird am Hochvakuum bei max. 60° das DMF abdestilliert. Der Rückstand wird in 5 ml Methylenchlorid gelöst, mit 1 ml wässriger Natriumbicarbonatlösung die organische Phase gewaschen und über Natriumsulfat getrocknet. Nach dem Eindampfen wird die rohe Titelverbindung durch Chromatographie (38 g Kieselgel 60, Laufmittel Essigester) gereinigt. FAB-MS: $(M+H)^+$ = 719; $R_f$ (R) = 0,7.

Beispiel 11: N-(2(R,S)-Benzyl-3-pivaloyl-propionyl)-Ser-Cha $\overset{C}{-}$ Val-Val-Tyr-OMe

Analog Beispiel 10 wird ein Diastereomerengemisch der Titelverbindung ausgehend von 80 mg H-Ser-Cha $\overset{C}{-}$ Val-Val-Tyr-OMe (Rohprodukt), 17,5 mg 2(R,S)-Benzyl-3-pivaloylpropionsäure, 12,8 mg HOBH und 15,8 mg DCCI in 0,64 ml DMF hergestellt. FAB-MS: $(M+H)^+$ = 865; $R_f$ (R) = 0,7; $t_{Ret}$(B) = 26,68 und 26,71 Min.

Das Ausgangsmaterial wird folgendermassen hergestellt:

a) H-Ser-Cha $\overset{C}{-}$ Val-Val-Tyr-OMe:

Analog Beispiel 10a) wird die Titelverbindung ausgehend von 95 mg Boc-Ser-Cha $\overset{C}{-}$ Val-Val-Tyr-OMe in 2,3 ml 95%iger Trifluoressigsäure hergestellt. $R_f$ (R) = 0.

b) BOC-Ser-Cha $\overset{C}{-}$ Val-Val-Tyr-OMe:

Analog Beispiel 10b) wird die Titelverbindung ausgehend von 109,5 mg H-Cha $\overset{C}{-}$ Val-Val-Tyr-OMe, 45,1 mg BOC-Ser-OH, 39,8 mg HOBH und 49,6 mg DCCI hergestellt und durch Chromatographie gereinigt. FAB-MS: $(M+H)^+$ = 735; $R_f$ (R) = 0,65.

Beispiel 12: N-(2(R,S)-Benzyl-3-pivaloyl-propionyl)-Phe-Cha $\overset{C}{-}$ Val-Val-Tyr-OMe

48,5 mg H-Phe-Cha $\overset{C}{-}$ Val-Val-Tyr-OMe und 20,8 mg 2(R,S)-Benzyl-3-pivaloyl-propionsäure werden in 2 ml DMF gelöst und mit 31,7 g BOP und 14,6 µl Triethylamin versetzt. Die Lösung wird bei Raumtemperatur für 15 Std. gerührt und danach eingedampft. Der Rückstand wird in wenig Diisopropylether digeriert. FAB-MS: $(M+H)^+$ = 925; $R_f$ (S) = 0,5; $t_{Ret}$(B) = 30,7 und 31,1 Min.

Das Ausgangsmaterial wird folgendermassen hergestellt:

a) H-Phe-Cha $\overset{C}{-}$ Val-Val-Tyr-OMe:

Eine Lösung aus 83 mg Fmoc-Phe-Cha $\overset{C}{-}$ Val-Val-Tyr-OMe, 2,4 ml Piperidin und 2,4 ml DMF wird für 90 Min. bei Raumtemperatur gerührt und danach eingedampft. Der Rückstand wird in wenig Methylenchlorid gelöst und mit Diisopropylether gefällt. Der Niederschlag wird bei 0° abfiltriert und anschliessend aus Dioxan/Wasser 9:1 lyophilisiert. FAB-MS: $(M+H)^+$ = 695; $R_f$ (Q) = 0,36.

b) Fmoc-Phe-Cha $\overset{C}{-}$ Val-Val-Tyr-OMe:

Eine Mischung aus 50 mg H-Cha $\overset{C}{-}$ Val-Val-Tyr-OMe, 67,3 mg Fmoc-Phe-OTcp, 17 µl N-Ethyldiisopropylamin und 1,2 ml DMF wird 75 Min. bei Raumtemperatur gerührt. Das Reaktionsgemisch wird eingedampft, und der Rückstand wird mit 5 ml Diisopropylether gefällt. Der Niederschlag wird abfiltriert und ein zweites Mal aus DMF/Diisopropylether gefällt. $R_f$ (Q) = 0,37.

Beispiel 13: N-(2(R,S)-Benzyl-3-pivaloyl-propionyl)-Leu-Cha $\overset{C}{-}$ Val-Val-Tyr-OMe

35,5 mg H-Leu-Cha $\stackrel{c}{-}$ Val-Val-Tyr-OMe und 16 mg 2(R,S)-Benzyl-3-pivaloyl-propionsäure werden in 2 ml DMF gelöst und mit 28,5 mg BOP und 11,2 µl Triethylamin versetzt. Nach 150 Min. Rühren bei Raumtemperatur wird eingeengt und der Rückstand in 20 ml Diisopropylether digeriert. FAB-MS: (M+H) + = 891; $R_f$ (Q) = 0,57; $t_{Ret}$(B) = 30,1 und 30,4 Min.

Das Ausgangsmaterial wird folgendermassen hergestellt:

a) H-Leu-Cha $\stackrel{c}{-}$ Val-Val-Tyr-OMe:

Eine Lösung aus 108 mg Fmoc-Leu-Cha $\stackrel{c}{-}$ Val-Val-Tyr-OMe, 3,2 ml Piperidin und 3,2 ml DMF wird für 60 Min. bei Raumtemperatur gerührt und danach eingedampft. Der Rückstand wird in wenig DMF gelöst und mit 20 ml Diisopropylether gefällt. Der Niederschlag wird bei 0° abfiltriert und anschliessend aus Dioxan/Wasser 9:1 lyophilisiert. FAB-MS: $(M+H)^+$ = 661; $R_f$ (Q) = 0,64.

b) Fmoc-Leu-Cha $\stackrel{c}{-}$ Val-Val-Tyr-OMe:

Eine Mischung aus 80 mg H-Cha $\stackrel{c}{-}$ Val-Val-Tyr-OMe, 101 mg Fmoc-Leu-OTcp, 27,5 µl N-Ethyldiisopropylamin und 1,4 ml DMF wird 90 Min. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert. $R_f$ (Q) = 0,375.

Beispiel 14: BBSP-Leu-Cha $\stackrel{c}{-}$ Val-Val-Tyr-OMe

35,5 mg H-Leu-Cha $\stackrel{c}{-}$ Val-Val-Tyr-OMe und 18,4 mg 2(S)-Benzyl-3-tert-butylsulfonylpropionsäure (BBSP-OH) werden in 1 ml DMF gelöst und mit 28,5 mg BOP und 11,2 µl Triethylamin versetzt. Nach 5 Std. Rühren bei Raumtemperatur wird eingeengt und der Rückstand in Diisopropylether digeriert. FAB-MS: $(M+H)^+$ = 927,5; $R_f$ (Q) = 0,54; $t_{Ret}$(B) = 28,0 Min.

Beispiel 15: BBSP-Leu-Cha $\stackrel{c}{-}$ Val-Val-p-biphenylylmethylamid

40 mg H-Leu-Cha $\stackrel{c}{-}$ Val-Val-p-biphenylylmethylamid und 18,4 mg BBSP-OH werden in 1 ml DMF gelöst und mit 28,5 mg BOP und 11 µl Triethylamin versetzt. Nach 5 Std. Rühren bei Raumtemperatur wird eingeengt und der Rückstand in Diisopropylether digeriert. FAB-MS: $(M+H)^+$ = 916; $R_f$ (Q) = 0,58.

Das Ausgangsmaterial wird folgendermassen hergestellt:

a) H-Leu-Cha $\stackrel{c}{-}$ Val-Val-p-biphenylylmethylamid:

Eine Lösung aus 108 mg Fmoc-Leu-Cha $\stackrel{c}{-}$ Val-Val-p-biphenylylmethylamid, 3 ml Piperidin und 3 ml DMF wird für 60 Min. bei Raumtemperatur gerührt und danach eingedampft. Der Rückstand wird in wenig DMF gelöst und mit 20 ml Diisopropylether gefällt. Der Niederschlag wird bei 0° abfiltriert und anschliessend aus Dioxan/Wasser 9:1 lyophilisiert. FAB-MS: $(M+H)^+$ = 649; $R_f$ (Q) = 0,72.

b) Fmoc-Leu-Cha $\stackrel{c}{-}$ Val-Val-p-biphenylylmethylamid:

Eine Mischung aus 100 mg H-Cha $\stackrel{c}{-}$ Val-Val-p-biphenylylmethylamid, 120 mg Fmoc-Leu-OTcp, 35 µl N-Ethyldiisopropylamin und 2 ml DMF wird 90 Min. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert. $R_f$ (Q) = 0,41.

c) H-Cha $\stackrel{c}{-}$ Val-Val-p-biphenylylmethylamid:

290 mg N-(5(S)-Azido-6-cyclohexyl-4(S)-hydroxy-2(S)-isopropylhexanoyl)-Val-p-biphenylylmethylamid

werden in 40 ml Methanol gelöst, mit 70 mg Palladiumkohle (5 % Pd) versetzt und mit Wasserstoff reduziert. Nach 2 Std. bei Raumtemperatur wird vom Katalysator abfiltriert, die Lösung eingeengt und der Rückstand gefällt. FAB-MS: $(M+H)^+$ = 536; $R_f$ (T) = 0,23.

d) N-(5(S)-Azido-6-cyclohexyl-4(S)-hydroxy-2(S)-isopropylhexanoyl)-Val-p-biphenylylmethylamid:

386 mg N-(5(S)-Azido-6-cyclohexyl-4(S)-tert-butyldimethylsilyloxy-2(S)-isopropyl-hexanoyl)-Val-p-biphenylylmethylamid in 5 ml DMF werden mit 360 mg Tetrabutylammoniumfluorid versetzt. Nach 4 Std. wird die Lösung eingeengt und der Rückstand in ca. 100 ml Essig ester aufgenommen und mit Natriumbicarbonatlösung gewaschen. Nach Einengen der Lösung wird der Rückstand aus Methylenchlorid/Hexan kristallisiert. FAB-MS: $(M+H)^+$ = 562; $R_f$ (U) = 0,12.

e) N-(5(S)-Azido-6-cyclohexyl-4(S)-tert-butyldimethylsilyloxy-2(S)-isopropyl-hexanoyl)-Val-p-biphenylylmethylamid:

376 mg 5(S)-Azido-6-cyclohexyl-4(S)-tert-butyldimethylsilyloxy-2(S)-isopropyl-hexansäure (J. Org. Chem. 54, 1178 (1989)) und 250 mg Valin-p-biphenylylmethylamid werden in 10 ml DMF gelöst und mit 404 mg BOP und 0,16 ml Triethylamin versetzt. Nach 16 Std. bei Raumtemperatur wird eingeengt, und der Rückstand mit Diisopropylether digeriert. FAB-MS: $(M+H)^+$ = 676; $R_f$ (U) = 0,45.

f) Valin-p-biphenylylmethylamid:

980 mg BOC-Val-p-biphenylylmethylamid werden in 10 ml Dioxan/4N HCl während 30 Min. bei Raumtemperatur stehen gelassen. Anschliessend wird eingeengt und zwischen Essigester und 1N NaOH verteilt. Die organische Phase wird eingeengt und der Rückstand an Kieselgel chromatographiert. $R_f$ (F) = 0,4.

g) BOC-Val-p-biphenylylmethylamid:

3,9 g N-tert-Butoxycarbonyl-L-valin-hydroxysuccinimidester und 2,7 g Biphenylylmethylamin werden in 150 ml Methylenchlorid bei Raumtemperatur während 1 Std. gerührt. Die Mischung wird durch Kieselgel filtriert und eingedampft, und der Rückstand aus Hexan kristallisiert. FAB-MS: (M+H) = 383; $R_f$ (U) = 0,28.

Beispiel 16: BBSP-Leu-Cha $\overset{C}{-}$ Val-5-phenylpentylamid

65 mg H-Leu-Cha $\overset{C}{-}$ Val-5-phenylpentylamid und 31 mg BBSP-OH werden in 1,5 ml DMF gelöst und mit 44 mg BOP und 18 µl Triethylamin versetzt. Nach 5 1/2 Std. Rühren bei Raumtemperatur wird das Lösungsmittel abgezogen und der Rückstand mit Diisopropylether digeriert. FAB-MS: $(M+H)^+$ = 797; $R_f$ - (Q) = 0,45.

Das Ausgangsmaterial wird folgendermassen hergestellt:

a) H-Leu-Cha $\overset{C}{-}$ Val-5-phenylpentylamid:

Eine Lösung aus 150 mg Fmoc-Leu-Cha $\overset{C}{-}$ Val-5-phenylpentylamid, 4 ml Piperidin und 4 ml DMF wird für 60 Min. bei Raumtemperatur gerührt und danach eingedampft. Der Rückstand wird in wenig DMF gelöst und mit 20 ml Diisopropylether gefällt. Der Niederschlag wird bei 0° abfiltriert und anschliessend aus Dioxan/Wasser 9:1 lyophilisiert. FAB-MS: $(M+H)^+$ = 530; $R_f$(Q) = 0,61.

b) Fmoc-Leu-Cha $\overset{C}{-}$ Val-5-phenylpentylamid:

Eine Mischung von 100 mg H-Cha $\overset{C}{-}$ Val-5-phenylpentylamid, 100 mg Fmoc-Leu-OTcp, 35 µl N-Ethyldiisopropylamin und 2 ml DMF wird 90 Min. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert. FAB-MS: $(M+H)^+$ = 739; $R_f(Q)$ = 0,39.

c) H-Cha $\overset{c}{-}$ Val-5-phenylpentylamid:

240 mg 5(S)-Azido-6-cyclohexyl-4(S)-hydroxy-2(S)-isopropyl-hexansäure-5-phenylpentylamid werden in 40 ml Mehanol gelöst, mit 70 mg Palladiumkohle (5 % Pd) versetzt und mit Wasserstoff reduziert. Nach 2 Std. bei Raumtemperatur wird vom Katalysator abfiltriert, die Lösung eingeengt und der Rückstand gefällt. FAB-MS: $(M+H)^+$ = 417; $R_f(T)$ = 0,19.

d) 5(S)-Azido-6-cyclohexyl-4(S)-hydroxy-2(S)-isopropyl-hexansäure-5-phenylpentylamid:

279 mg 5(S)-Azido-6-cyclohexyl-2(S)-isopropyl-4(S)-hexanolid (J. Org. Chem. 54, 1178 (1989)) werden in 816 mg 5-Phenylpentylamin während 3 Std. auf 60° erwärmt. Anschliessend wird das Gemisch an Kieselgel chromatographiert. FAB-MS: $(M+H)^+$ = 443; $R_f(V)$ = 0,4.

Beispiel 17: BBSP-Val-Leu $\overset{c}{-}$ Val-Val-Tyr-OMe

Analog Beispiel 7 wird die Titelverbindung ausgehend von 30 mg HCl•H-Val-Leu $\overset{c}{-}$ Val-Val-Tyr-OMe, 40 mg 2(S)-Benzyl-3-tert-butylsulfonylpropionsäure (BBSP-OH), 53 mg HBTU und 40 µl Triethylamin hergestellt, durch Flash-Chromatographie an Kieselgel (Laufmittel P) gereinigt und aus Dioxan/tert-Butanol/Wasser lyophilisiert.

Das Ausgangsmaterial wird folgendermassen hergestellt:

a) HCl•H-Val-Leu $\overset{c}{-}$ Val-Val-Tyr-OMe

wird ausgehend von 55 mg BOC-Val-Leu $\overset{c}{-}$ Val-Val-Tyr-OMe und 5 ml 4N HCl in Dioxan analog Beispiel 11) hergestellt. Das Produkt wird aus Dioxan lyophilisiert.

b) Boc-Val-Leu $\overset{c}{-}$ Val-Val-Tyr-OMe

wird analog Beispiel 7b) aus 60 mg H-Leu $\overset{c}{-}$ Val-Val-Tyr-OMe, 77 mg BOC-Val-OH, 135 mg HBTU und 100 µl Triethylamin hergestellt.

c) H-Leu $\overset{c}{-}$ Val-Val-Tyr-OMe

wird analog Beispiel 1j) aus 90 mg Z-Leu $\overset{cx}{-}$ Val-Val-Tyr-OMe durch katalytische Hydrierung in 10 ml Methanol/Wasser 9:1 und 15 mg Palladiumkohle (10 % Pd) erhalten.

d) Z-Leu $\overset{cx}{-}$ Val-Val-Tyr-OMe:

Analog Beispiel 1i) werden 150 mg Z-Leu $\overset{cx}{-}$ Val-OH (Herstellung nach EP 143 746) mit 115 mg H-Val-Tyr-OMe, 85 mg DCCI, 62 mg HOBt und 45 µl N-Methylmorpholin umgesetzt. Man erhält die Titelverbindung nach Flash-Chromatographie (Laufmittel P).

Beispiel 18: BBSP-Val-Sta-Val-Tyr-OH

An 260 mg (0,11 mMol) Fmoc-Tyr(OtBu)-p-benzyloxybenzylester-polystyrol-harz (1 % vernetzt) (Novabiochem, Läufelfingen, Schweiz), wird auf einer Fritte zur alternierenden Abspaltung des Fmoc-Schutzgruppe und zur Ankupplung der Säurederivate der im folgenden beschriebene Prozess repetitiv angewandt. Die Waschoperationen mit je ca. 3 ml Lösungsmittel und die Reaktionsoperationen erfolgen unter Schütteln.

In der ersten Stufe wird Fmoc-Val-OH wie folgt angekoppelt:

| Schritt Nr. | Wiederholung | Dauer | Operation |
|---|---|---|---|
| 1 | 7 mal | 2 Min. | Abspaltung der Fmoc-Schutzgruppe mit Piperidin/DMA 80:20 (v/v) |
| 2 | 2 mal | 1 Min. | Waschen mit DMA |
| 3 | 1 mal | 30 Min. | Voraktivierung für Kupplung: 3 Aeq. Fmoc-Val-OH, 3,3 Aeq. 0,5 M HOBt in DMA, 3,3 Aeq. 2,0 M Diisopropylcarbodiimid in DMA |
| 4 | 1 mal | 1 Std. | Kupplung bei Raumtemperatur nach Zugabe des vorher hergestellten Kupplungsgemisches zum Harz |
| 5 | 2 mal | 1 Min. | Waschen mit DMA |
| 6 | 1 mal | 5 Min. | Acetylieren der nicht umgesetzten Aminogruppen mit Acetanhydrid/Pyridin/DMA 10:10:80 (v/v) |
| 7 | 3 mal | 1 Min. | Waschen mit DMA |
| 8 | 2 mal | 1 Min. | Waschen mit Isopropanol |
| 9 | 2 mal | 1 Min. | Waschen mit DMA |

In Analogie dazu wird in der zweiten Stufe Fmoc-Sta-OH mit einer Reaktionszeit von 4 Std. in Schritt 3 angekoppelt; die Schritte 5 und 6 werden weggelassen. Die Ankopplung von Fmoc-Val-OH in der dritten Stufe und von 2(S)-Benzyl-3-tert-butylsulfonylpropionsäure (BBSP-OH) in der vierten Stufe erfolgen wiederum mit einer Reaktionszeit von 1 Std. in Schritt 3 und ohne die Schritte 5 und 6.

Das so erhaltene belegte Peptidsyntheseharz wird 2 mal während je 5 Min. mit je 3 ml Trifluoressigsäure/Wasser/1,2-Ethandithiol 95:5:2 (v/v) bei Raumtemperatur geschüttelt, abfiltriert und anschliessend 2 mal mit je 3 ml 1,2-Dichlorethan und 2 mal mit je 3 ml Trifluorethanol gewaschen. Die Filtrate werden im Vakuum eingedampft. Zur vollständigen Abspaltung der Schutzgruppen wird der Rückstand in 2 ml 95%iger wässriger Trifluoressigsäure gelöst und nach 1 Std. im Vakuum eingedampft. Der Rückstand wird erneut in 2 ml 95%iger Trifluoressigsäure gelöst, und das Peptid durch Zutropfen von Diisopropylether gefällt. Die Fällung wird in 1,2-Dichlorethan gelöst und durch Zugabe von Diisopropylether erneut gefällt. Durch Lyophilisation aus Dioxan/tert-Butanol/Wasser erhält man die Titelverbindung. FAB-MS: $(M+H)^+$ = 803; = $R_f(O)$ = 0,43; $t_{Ret}(B)$ = 22,0 Min.

Beispiel 19: BBSP-Val-Sta-Val-Tyr-OMe

Eine Lösung von 3 mg BBSP-Val-Sta-Val-Tyr-OH in 3 ml 1N HCl in Methanol wird 1 Std. bei Raumtemperatur belassen und anschliessend eingedampft. Lyophilisation des Rückstandes aus Dioxan ergibt die Titelverbindung FAB-MS: $(M+H)^+$ = 817; $R_f(O)$ = 0,75; $t_{Ret}(B)$ = 24,1 Min.

Beispiel 20: BBSP-Val-ACHPA-Val-Tyr-OH

An 240 mg (0,095 mMol) Fmoc-Tyr(OtBu)-p-benzyloxybenzylester-polystyrolharz (1 % vernetzt) werden analog Beispiel 18 die folgenden Säurederivate angekoppelt:

1. Fmoc-Val-OH
2. Fmoc-ACHPA-OH, 4 Std. Reaktionszeit, ohne Schritte 5, 6
3. Fmoc-Val-OH, ohne Schritte 5, 6
4. BBSP-OH, ohne Schritte 5, 6.

Das Peptid wird wie in Beispiel 18 vom Harz abgelöst und zweimal aus DMF durch Zutropfen von Diisopropylether bei 0° gefällt. Lyophilisation aus Dioxan/tert-Butanol/Wasser ergibt die Titelverbindung. FAB-MS: $(M+H)^+$ = 843; $R_f(O)$ = 0,40; $t_{Ret}(B)$ = 27,6 Min.

Das Ausgangsmaterial wird folgendermassen hergestellt:

a) Fmoc-ACHPA-OH:

Eine Lösung von 660 mg BOC-ACHPA-OH (J. Med. Chem. 28, 1779 (1985)) in 25 ml 4N HCl in Dioxan wird 30 Min. bei Raumtemperatur gerührt. Nach Verdünnen mit 10 ml Dioxan und Lyophilisation erhält man HCl•H-ACHPA-OH. Eine Lösung von 390 mg dieser Verbindung in 10 ml Dioxan/THF/Wasser 1:1:1 (v/v) wird mit 247 mg Natriumbicarbonat und 545 mg Fmoc-N-hydroxysuccinimid versetzt. Die trübe Mischung wird über Nacht bei Raumtemperatur gerührt, mit 2N HCl auf pH 2 angesäuert und auf 5 ml eingeengt. Das Konzentrat wird zwischen 0,1N HCl und Methylenchlorid verteilt, die organischen Phasen durch Watte filtriert und eingedampft. Der ölige Rückstand wird durch Flash-Chromatographie an 50 g Kieselgel (Laufmittel P) gereinigt und die produkthaltigen Fraktionen eingedampft. Kristallisation des erhaltenen Oels aus Essigester/Hexan ergibt die Titelverbindung. Smp. = 144°; $R_f(P)$ = 0,33; $t_{Ret}(B)$ = 27,9 Min.

Beispiel 21: BBSP-Phe-Sta-Val-Tyr-OH

An 300 mg (0,11 mMol) Fmoc-Tyr(OtBu)-p-benzyloxybenzylester-polystyrolharz (1 % vernetzt) werden analog Beispiel 18 die folgenden Säurederivate angekoppelt:

1. Fmoc-Val-OH
2. Fmoc-Sta-OH, 4 Std. Reaktionszeit, ohne Schritte 5, 6
3. Fmoc-Phe-OH, ohne Schritte 5, 6
4. BBSP-OH, ohne Schritte 5, 6.

Das Peptid wird wie in Beispiel 18 beschrieben vom Harz abgelöst und aus DMF durch Zutropfen von Diisopropylether gefällt. Die Fällung wird durch präparative HPLC aufgetrennt ($C_{18}$ Nucleosil® 5μ, 25 x 2 cm, Wasser/Acetonitril 60:40 mit je 0,1 % Trifluoressigsäure, 600 bar) und die produkthaltigen Fraktionen im Vakuum auf die Hälfte eingeengt. Nach Lyophilisation des Restvolumens erhält man die Titelverbindung. FAB-MS: $(M+H)^+$ = 851; = $R_f(P)$ 0,24; $t_{Ret}(B)$ = 23,9 Min.

Beispiel 22: BBSP-Val-Phe $\xrightarrow{red}$ Pro-Val-Tyr-OH

An 240 mg (0,095 mMol) Fmoc-Tyr(OtBu)-p-benzyloxybenzylester-polystyrolharz (1 % vernetzt) werden analog Beispiel 18 die folgenden Säurederivate angekoppelt:

1. Fmoc-Val-OH
2. Fmoc-Phe $\xrightarrow{red}$ Pro-OH, 4 Std. Reaktionszeit, ohne Schritte 5, 6
3. Fmoc-Val-OH, ohne Schritte 5, 6
4. BBSP-OH, ohne Schritte 5, 6.

Das Peptid wird wie in Beispiel 18 vom Harz abgelöst und zweimal mit Diisopropylether/Petrolether 1:1 gefällt. Lyophilisation aus ter-Butanol/Eisessig 9:1 ergibt die Titelverbindung; FAB-MS: $(M+H)^+$ = 885; $t_{Ret}(C)$ = 18,9 Min.

Das Ausgangsmaterial wird folgendermassen hergestellt:

a) N-BOC-L-phenylalaninol:

18,2 g L-Phenylalaninol und 28,4 g Di-tert-butyl-dicarbonat werden in 500 ml $CH_2Cl_2$ bei Raumtemperatur 2 Std. umgesetzt. Anschliessend wird das $CH_2Cl_2$ am Wasserstrahlvakuum abdestilliert und die kristalline Titelverbindung aus Ether umkristallisiert. Smp. 93-94°.

b) N-BOC-L-phenylalaninal:

Zu einer Lösung von 5,64 ml Oxalylchlorid in 100 ml $CH_2Cl_2$ wird bei -60° eine Lösung von 9,12 ml DMSO in 100 ml $CH_2Cl_2$ getropft. Nach vollständiger Zugabe lässt man noch 15 Min. ausreagieren und gibt anschliessend eine Lösung von 15,06 g N-BOC-L-Phenylalaninol in 200 ml $CH_2Cl_2$ zu. Nach 1 Std. bei -60° wird das Reaktionsgemisch auf Eis gegossen und mit 1N HCl die wässrige Phase auf pH 3 gestellt. Die organische Phase wird je einmal mit eiskalter wässriger $NaHCO_3$-Lösung und mit Wasser gewaschen, über $MgSO_4$ getrocknet und bei Raumtemperatur im Vakuum eingedampft. Die zurückbleibende Titelverbindung wird aus Hexan umkristallisiert und im Tiefkühlschrank wegen Racemisierungsgefahr gelagert. Smp. 74-76°; $[\alpha]_D^{20}$ = -38,1° (c = 1,09, $CH_3OH$).

c) BOC-Phe $\xrightarrow{red}$ Pro-OMe:

12,0 g N-BOC-L-phenylalaninal und 45,5 g L-Prolinmethylester-hydrochlorid werden in 400 ml Methanol gelöst und mit 2,36 g Natriumcyanoborhydrid (85 %) bei Raumtemperatur umgesetzt. Nach 6 Std. Rühren bei Raumtemperatur wird am Wasserstrahlvakuum das Lösungsmittel abdestilliert und die rohe Titelverbindung durch Flash-Chromatographie an 600 g Kieselgel 60 (Laufmittel A) gereinigt. Gelbes, viskoses Oel, $R_f$-(A) = 0,65. Laut $^1$H-NMR ($CDCl_3$) liegt eine ca. 9:1-Mischung von Diastereomeren vor, herrührend von nicht 100 % enantiomerenreinem eingesetztem N-BOC-L-phenylalaninal.

d) BOC-Phe $\xrightarrow{red}$ Pro-OH:

6,18 g BOC-Phe $\xrightarrow{red}$ Pro-OMe werden in 70 ml Methanol/Wasser 4:1 gelöst und über Nacht bei Raumtemperatur mit 18,7 ml 1N NaOH verseift. Anschliessend wird am Vakuum das Methanol abdestilliert und unter Eis-Kühlung mit ca. 15 ml 1N HCl auf pH 9 gestellt. Essigesterextraktion ergibt nach Trocknung über Natriumsulfat die kristalline, rohe Titelverbindung. Nach Digerieren in wenig kaltem Essigester kann die reine Titelverbindung erhalten werden. Smp. 160-166°; $R_f$(V) = 0,35.

e) H-Phe $\xrightarrow{red}$ Pro-OH•2HCl:

696,8 mg BOC-Phe $\xrightarrow{red}$ Pro-OH werden in 28 ml 4,25N Salzsäure in Dioxan bei Raumtemperatur 30 Min. gerührt. Anschliessend wird am Vakuum eingedampft und die Titelverbindung als weisses Pulver isoliert und direkt weiterverarbeitet.

f) Fmoc-Phe $\xrightarrow{red}$ Pro-OH:

186 mg H-Phe $\xrightarrow{red}$ Pro-OH•2HCl (Rohprodukt) und 133,5 mg Fmoc-N-hydroxysuccinimidester werden in 4 ml Wasser mit 0,166 ml Triethylamin versetzt und 4 Std. bei Raumtemperatur gerührt. Das Reaktionsgemisch wird mit Essigester versetzt und die wässrige Phase mit 1N wässriger HCl auf pH 4-6 gestellt. Die organische Phase wird abgetrennt, über Natriumsulfat getrocknet und durch Eindampfen am Wasserstrahlvakuum die rohe Titelverbindung erhalten. Durch Chromatographie an 7 g Kieselgel 60 (Laufmittel Essigester: Methanol = 4:1) wird die reine Verbindung erhalten. $R_f$(V) = 0,4; FAB-MS: $(M+H)^+$ = 471; $[\alpha]_D^{20}$ = -27,1° (c = 0,634, $CHCl_3$).

Beispiel 23: BBSP-Val-Phe $\xrightarrow{red}$ Pro-Val-Tyr-OMe

Analog Beispiel 1 wird die Titelverbindung ausgehend von 65 mg H-Val-Phe $\xrightarrow{red}$ Pro-Val-Tyr-OMe (Rohprodukt), 32,4 mg 2(S)-Benzyl-3-tert-butylsulfonylpropionsäure (BBSP-OH), 17,5 mg HOBt, 25,8 mg DCCI und 21 mg N-Methylmorpholin in 16 ml DMF hergestellt. $R_f$(R) = 0,55; FAB-MS: $(M+H)^+$ = 890; $t_{Ret}$-(B) = 23,4 Min.

Das Ausgangsmaterial wird wie folgt hergestellt:

a) H=Val-Phe $\overset{red}{\overline{\quad\quad}}$ Pro-Val-Tyr-OMe:

180 mg BOC-Val-Phe $\overset{red}{\overline{\quad\quad}}$ Pro-Val-Tyr-OMe werden bei 0° 90 Min. mit 5 ml 95%iger wässriger Trifluoressigsäure behandelt. Anschliessend wird mit $CH_2Cl_2$ und festem $NaHCO_3$ aufgearbeitet. Die organische Phase wird über Hyflo® filtriert und anschliessend mit wenig Wasser gewaschen. Nach Trocknen über $MgSO_4$ und Verdampfen des Lösungsmittels wird die Titelverbindung erhalten. FAB-MS: $(M+H)^+$ = 624.

b) BOC-Val-Phe $\overset{red}{\overline{\quad\quad}}$ Pro-Val-Tyr-OMe

wird ausgehend von 39 mg H-Phe $\overset{red}{\overline{\quad\quad}}$ Pro-Val-Tyr-OMe, 17,9 mg BOC-Val-OH, 14,7 mg HOBH und 18,4 mg DCCI analog Beispiel 1 hergestellt und durch Chromatographie an Kieselgel 60 (Laufmittel R) gereinigt. $R_f(R)$ = 0,5; FAB-MS: $(M+H)^+$ = 724.

c) H-Phe $\overset{red}{\overline{\quad\quad}}$ Pro-Val-Tyr-OMe

wird ausgehend von 300 mg BOC-Phe $\overset{red}{\overline{\quad\quad}}$ Pro-Val-Tyr-OMe und 6 ml 95%iger wässriger Trifluoressigsäure analog Beispiel 23a hergestellt und durch Fällen aus Ether/Hexan isoliert. FAB-MS: $(M+H)^+$ = 525.

d) BOC-Phe $\overset{red}{\overline{\quad\quad}}$ Pro-Val-Tyr-OMe

wird ausgehend von 348 mg BOC-Phe $\overset{red}{\overline{\quad\quad}}$ Pro-OH, 294,3 mg H-Val-Tyr-OMe (Beispiel 1h), 183,6 mg HOBt und 247,6 mg DCCI in 40 ml DMF bei Raumtemperatur über Nacht hergestellt. Zur Aufarbeitung wird am Hochvakuum bei 60° das DMF grösstenteils abdestilliert, der Rückstand bei 0° mit 0,5 ml Essigsäure/$H_2O$ 1:1 versetzt und 30 Min. gerührt. Die gelbe Suspension wird über Hyflo® filtriert, mit wenig DMF nachgewaschen und das Filtrat am Hochvakuum konzentriert. Dieses wird in 100 ml $CH_2Cl_2$ aufgenommen, mit 10 ml Wasser versetzt und die Wasserphase mit 1N NaOH auf pH 9 gestellt. Nach Abtrennen der organischen Phase und einmaligem Waschen mit wenig Wasser wird diese über Natriumsulfat getrocknet und daraus durch Eindampfen die rohe Titelverbindung isoliert. Säulenchromatographie an Kieselgel 60 (Laufmittel W) ergibt die reine Titelverbindung, die aus Toluol/Hexan umkristallisiert werden kann. Smp. 173-175°; FAB-MS: $(M+H)^+$ = 625; $R_f(W)$ = 0,3.

Beispiel 24: BBSP-Val-Cha $\overset{red}{\overline{\quad\quad}}$ Pro-Val-Tyr-OMe

Analog Beispiel 1 wird die Titelverbindung ausgehend von 172 mg H-Val-Cha $\overset{red}{\overline{\quad\quad}}$ Pro-Val-Tyr-OMe (Rohprodukt), 85,4 mg BBSP-OH, 46 mg HOBt, 67,6 mg DCCI und 55,2 mg N-Methylmorpholin in 15 ml DMF hergestellt. $R_f(R)$ = 0,5; FAB-MS: $(M+H)^+$ = 896; $t_{Ret}(B)$ = 24,7 Min.

Das Ausgangsmaterial wird wie folgt erhalten:

a) H-Val-Cha $\overset{red}{\overline{\quad\quad}}$ Pro-Val-Tyr-OMe:

280 mg BOC-Val-Cha $\overset{red}{\overline{\quad\quad}}$ Pro-Val-Tyr-OMe werden analog Beispiel 23a) mit 5 ml 95%iger wässriger Trifluoressigsäure behandelt und als Rohprodukt weiter verarbeitet.

b)BOC-Val-Cha $\overset{red}{\overline{\quad\quad}}$ Pro-Val-Tyr-OMe:

Analog Beispiel 1 wird die Titelverbindung ausgehend von 1,06 g H-Cha $\overset{red}{\overline{\quad\quad}}$ Pro-Val-Tyr-OMe (Rohprodukt), 476 mg BOC-Val-OH, 395 mg HOBH und 496 mg DCCI in 25 ml DMF hergestellt und durch Chromatographie an Kieselgel 60 (Laufmittel Essigester) gereinigt. $R_f(R)$ = 0,65; IR($CH_2Cl_2$): 2920, 1735, 1665 $cm^{-1}$.

c) H-Cha $\overset{red}{\overline{\quad\quad}}$ Pro-Val-Tyr-OMe:

1,129 g BOC-Cha $\overset{red}{\overline{\quad\quad}}$ Pro-Val-Tyr-OMe werden analog Beispiel 23a) mit 20 ml 95%iger wässriger

Trifluoressigsäure behandelt und als Rohprodukt weiter verarbeitet.

d) BOC-Cha $\xrightarrow{red}$ Pro-Val-Tyr-OMe:

Analog Beispiel 1 wird die Titelverbindung ausgehend von 769 mg BOC-Cha $\xrightarrow{red}$ Pro-OH, 639 mg H-Val-Tyr-OMe, 398 mg HOBt und 537 mg DCCI in 100 ml DMF hergestellt und durch Chromatographie an Kieselgel 60 (Laufmittel Methanol) gereinigt. $R_f$(MeOH) = 0,88; IR($CH_2Cl_2$): 2920, 1740, 1675 $cm^{-1}$.

e) BOC-Cha $\xrightarrow{red}$ Pro-OH:

2,09 g BOC-Phe $\xrightarrow{red}$ Pro-OH werden in 40 ml THF mit 2,27 ml methanolischer HCl und 0,4 g Nishimura-Katalysator bei Normaldruck hydriert. Anschliessend wird über Hyflo® filtriert, mit wenig Methanol/THF der Filterrückstand gewaschen und das Filtrat mit Essigester und wässriger Natriumbicarbonatlösung versetzt. Der pH der wässrigen Phase wird auf 9 gestellt, die organische Phase abgetrennt, über Natriumsulfat getrocknet und am Vakuum eingeengt. Umkristallisation des Rückstandes aus Essigester/Hexan ergibt die reine Titelverbindung. Smp. 155-158°; FAB-MS: $(M+H)^+$ = 355; $[\alpha]_D^{20}$ = -25° (c = 1,07, $CHCl_3$).

Beispiel 25: BBSP-Val-Cha $\xrightarrow{red}$ Val-Val-Tyr-OH

An 260 mg (0,11 mMol) Fmoc-Tyr(OtBu)-p-benzyloxybenzylester-polystyrolharz (1 % vernetzt) werden analog Beispiel 18 die folgenden Säurederivate angekoppelt:

1: Fmoc-Val-OH

2: Fmoc-Cha $\xrightarrow{red}$ Val-OH, 4 Std. Reaktionszeit, ohne Schritte 5, 6

3: Fmoc-Val-OH, ohne Schritte 5, 6

4: BBSP-OH, ohne Schritte 5, 6.

Das Peptid wird wie in Beispiel 18 vom Harz abgelöst und zweimal mit Diisopropylether/Petrolether 1:1 gefällt. Lyophilisation aus tert-Butanol ergibt die Titelverbindung; FAB-MS: $(M+H)^+$ = 877; $t_{Ret}(C)$ = 17,75 Min.

Das Ausgangsmaterial wird folgendermassen hergestellt:

a) Fmoc-Cha $\xrightarrow{red}$ Val-OH

wird aus 1,71 g H-Cha $\xrightarrow{red}$ Val-OH•2HCl (Rohprodukt) und 1,94 g Fmoc-N-hydroxysuccinimidester in 100 ml Wasser und 2,3 ml Triethylamin analog Beispiel 22f) hergestellt. Smp. (Methanol) 188° (Zersetzung); FAB-MS: $(M+H)^+$ = 479.

b) H-Cha $\xrightarrow{red}$ Val-OH•2HCl

wird aus 1,85 g BOC-Cha $\xrightarrow{red}$ Val-OH in 75 ml 4,25N Salzsäure in Dioxan analog Beispiel 22e) hergestellt und roh weiterverwendet.

c) BOC-Cha $\xrightarrow{red}$ Val-OH

wird aus 540 mg BOC-Phe $\xrightarrow{red}$ Val-OH, 0,5 g Nishimura-Katalysator und 3,78 ml methanolischer HCl in 70 ml Methanol analog Beispiel 24e) hergestellt. Smp. 187° (Zersetzung); FAB-MS: $(M+H)^+$ = 357; IR-(KBr): 3380, 2910, 1690 $cm^{-1}$.

d) BOC-Phe $\xrightarrow{red}$ Val-OH

wird aus 730 mg BOC-Phe $\xrightarrow{red}$ Val-OMe und 2,2 ml 1N NaOH in 8 ml Methanol/Wasser 4:1 analog Beispiel 22d) hergestellt. Smp. 207° (Zersetzung); FAB-MS: $(M+H)^+$ = 351; IR(KBr): 3365, 2970, 1690 $cm^{-1}$.

e) BOC-Phe $\xrightarrow{red}$ Val-OMe

wird aus 3,7 g N-BOC-L-phenylalaninal, 14,8 g L-Valin-methylester-hydrochlorid und 770 mg Natriumcyanoborhydrid (85 %) in 150 ml Methanol analog Beispiel 22c) hergestellt. Smp. 74-76° (Ether/Pentan); $[\alpha]_D^{20}$ = -17,5° (c = 0,85, $CHCl_3$).

Beispiel 26: Gelatine-Lösung

Eine sterilfiltrierte wässrige Lösung von Z-Arg-Arg-Pro-Phe-Val-Cha $\overset{c}{-}$ Val-Val-Tyr-OMe wird unter Erwärmen mit einer sterilen Gelatinelösung, welche als Konservierungsmittel Phenol enthält, unter aseptischen Bedingungen so vermischt, dass 1,0 ml Lösung folgende Zusammensetzungen hat:

| | |
|---|---|
| Z-Arg-Arg-Pro-Phe-Val-Cha $\overset{c}{-}$ Val-Val-Tyr--OMe | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 27: Sterile Trockensubstanz zur Injektion

Man löst 5 mg Z-Arg-Arg-Pro-Phe-Val-Cha $\overset{c}{-}$ Val-Val-Tyr-OMe in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 28: Nasenspray

In einer Mischung von 3,5 ml Myglyol 812® und 0,08 g Benzylalkohol werden 500 mg fein gemahlenes (<5,0 µm) BBSP-Val-Cha $\overset{c}{-}$ Val-Val-Tyr-OMe suspendiert. Diese Suspension wird in einen Behälter mit Dosierventil eingefüllt. Es werden 5,0 g Freon® 12 unter Druck durch das Ventil in den Behälter abgefüllt. Durch Schütteln wird das Freon® in der Myglyol-Benzylalkoholmischung gelöst. Dieser Spraybehälter enthält ca. 100 Einzeldosen, die einzeln appliziert werden können.

Beispiel 29: Lacktabletten

Für die Herstellung von 10 000 Tabletten enthaltend je 100 mg Wirkstoff werden folgende Bestandteile verarbeitet:

| | |
|---|---|
| BBSP-Val-Cha $\overset{c}{-}$ Val-Val-Tyr--OMe | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q.s. |

Ein Gemisch des BBSP-Val-Cha $\overset{c}{-}$ Val-Val-Tyr-OMe, 50 g Maisstärke und der kolloidalen Kieselsäure

wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45° während 30 Min. im Wirbelschichttrockner getrocknet. Das trockene Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid durch gleichmässiges Aufsprühen während 30 Min. überzogen; dabei wird durch gleichzeitiges Einblasen von Luft von 60° getrocknet.

Anstelle der in den Beispielen 26-29 genannten Wirkstoffe kann man in diesen Beispielen auch dieselbe Menge eines anderen Wirkstoffes der vorangehenden Beispiele verwenden.

## Beispiel 30: Hemmung der isolierten HIV-1 gag-Protease

10 µl einer Lösung von HIV-1 gag-Protease (Aceton-Extrakt einer in E. coli exprimierten gag-Protease gemäss J. Hansen et al., The EMBO Journal 7, 1785 (1988)) und 190 µl β-Morpholinoethansulfonsäure-Pufferlösung pH 6 enthaltend 0,01 % 2-Amino-4-nitrophenol als internem Standard werden bei 37° vorinkubiert. Dann werden gleichzeitig 10 µl einer 0,24 mM DMSO-Lösung des Substrats H-Arg-Arg-Ser-Asn-Gln-Val-Ser-Gln-Asn-Tyr-Pro- Ile-Val-Gln-Asn-Ile-Gln-Gly-Arg-Arg-OH (Icosapeptid gemäss J. Schneider et al., Cell 54, 363 (1988), hergestellt auf einem automatisierten Peptidsynthesegerät mit Fmoc-geschützten Aminosäurebausteinen) und 10 µl einer DMSO-Lösung der auf Hemmwirkung zu untersuchenden Verbindung in einer Konzentration von 22 x $10^{-4}$M oder 22 x $10^{-6}$M zugegeben. Nach einer Stunde werden 50 µl Reaktionslösung entnommen, mit 5 µl 0,3 M Perchlorsäure versetzt und zentrifugiert. Die Menge an unverbrauchtem Substrat und die Spaltprodukte in der überstehenden Lösung werden mit HPLC bestimmt und daraus die prozentuale Hemmung bei $10^{-4}$M und $10^{-6}$M berechnet.

Für die HPLC-Analyse wird eine 125 x 4,6 mm reversed phase $C_{18}$ Nucleosil® 5 µ Säule verwendet; Gradient 10 % Acetonitril/0,1 % Trifluoressigsäure in Wasser → 25 % Acetonitril/0,08 % Trifluoressigsäure in Wasser in 30 Min., Durchflussrate 1,5 ml/min.

Die Verbindungen der meisten vorstehenden Beispiele weisen bei Konzentrationen von $10^{-4}$M eine Hemmwirkung von über 80 % und bei $10^{-6}$M eine Hemmwirkung von über 50 % auf.

## Beispiel 31: Schutz gegen HIV-Infektion im Zelltest

Die verwendete Zellinie MT-2 ist eine menschliche T-Zell-Leukämie, die mit HTLV-1 transformiert ist und HTLV-1 kontinuierlich produziert, was die Zellen extrem empfindlich für den zytopathogenen Effekt von HIV macht (Science 229, 563 (1985)). Die MT-2 Zellen werden in RPMI 1640 Medium enthaltend 12 % hitze-inaktiviertes fötales Kälberserum (Seromed Biochrom KG, Berlin, BRD), Glutamin und Standard-Antibiotika kultiviert. Die Zellen werden bei 37° in einer befeuchteten Atmosphäre von 5 % $CO_2$ in Luft gehalten und in der logarithmischen Wachstumsphase für den Zelltest verwendet.

HIV LAV-03 (AIDS Research and Reference Reagent Program, NIH, Bethesda, MD, USA) wird in A 3.01-Zellen gezüchtet. Der Titer wird im Reverse Transcriptase Assay bestimmt. Für die verwendete Charge beträgt er 2 x $10^7$ IU/ml. 40'000 exponentiell wachsende MT-2 Zellen in 50 µl Kulturmedium werden in jede der Vertiefungen einer 96 Rundboden-Titerplatte gegeben. Die zu untersuchenden Verbindungen werden in vorgegebener Konzentration in 50 µl Kulturmedium beigefügt, und unmittelbar danach HIV in 100 µl Kulturmedium zugegeben. Zu Vergleichsproben wird 100 µl Kulturmedium ohne HIV zugegeben. Die Titerplatten werden sechs Tage inkubiert. Danach wird die Lebensfähigkeit der HIV-infizierten und der Vergleichs-Zellen im MTT-Assay geprüft: Der MTT-Assay beruht auf der Reduktion von gelbgefärbtem 3-(4,5-dimethylthiazol-2-yl)-2,5-diphenyltetrazolium-bromid (MTT, Sigma, St.Louis, USA) durch mytochondrische Dehydrogenasen von metabolisch aktiven Zellen zu einem blauen Formazan, das spektrophotometrisch bei 540 nm gemessen werden kann (J. Virological Methods 20, 309 (1988)). Die Lebensfähigkeit von Vergleichszellen und HIV-infizierten Zellen wird ebenfalls mikroskopisch in einem Hämozytometer nach der Trypanblau-Ausschlussmethode bestimmt.

Die untersuchten Verbindungen der vorstehenden Beispiele weisen bei Konzentrationen von $10^{-5}$ Mol/l Schutzwirkung gegen HIV-Infektion auf.

## Ansprüche

1. Verwendung von Verbindungen vom Typus der Renininhibitoren oder verwandter Aspartatproteinaseinhibitoren zur Herstellung von pharmazeutischen Präparaten zur Anwendung als gag-Protease-Hemmer.

2. Verwendung von Verbindungen vom Typus der Renininhibitoren oder verwandter Aspartatproteinaseinhibitoren zur Herstellung von pharmazeutischen Präparaten zur Bekämpfung von durch Retroviren verursachten Krankheiten.

3. Verwendung gemäss Anspruch 1 oder 2, dadurch gekennzeichnet, dass Verbindungen verwendet werden, die gegenüber der gag-Protease als isoliertes Enzym in Konzentrationen von $10^{-6}$ Mol/l oder weniger oder gegenüber der gag-Protease in der Zelle in Konzentrationen von $10^{-5}$ Mol/l oder weniger Hemmwirkungen von 50 % oder mehr zeigen.

4. Verwendung gemäss einem der Ansprüche 1, 2 oder 3, dadurch gekennzeichnet, dass Verbindungen der Formel

$R^2$ - AAN - Q - AAC - $R^1$ (I),

worin AAN ein bivalentes Radikal bestehend aus einem bis fünf bivalenten $\alpha$-Aminosäureresten bedeutet, welches N-terminal mit dem Rest $R^2$ und C-terminal mit dem Radikal Q verbunden ist, AAC eine Bindung oder ein bivalentes Radikal bestehend aus einem bis fünf bivalenten $\alpha$-Aminosäureresten bedeutet, welches N-terminal mit dem Radikal Q und C-terminal mit dem Rest $R^1$ verbunden ist, $R^1$ für Hydroxy, verethertes Hydroxy, Amino oder substituiertes Amino mit Ausnahme eines von einer $\alpha$-Aminosäure abgeleiteten Aminorestes steht, $R^2$ Wasserstoff oder einen Acylrest mit Ausnahme eines gegebenenfalls N-substituierten Acylrestes einer natürlichen Aminosäure bedeutet, und Q einen bivalenten Rest bedeutet, der ein Isoster eines Dipeptides ist, oder Salze von solchen Verbindungen verwendet werden.

5. Verwendung gemäss Anspruch 4, dadurch gekennzeichnet, dass in einer Verbindung der Formel I Q für eine Teilstruktur ausgewählt unter den Formeln

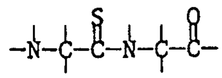 (IIa)

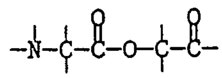 (IIb)

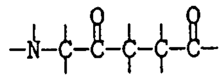 (IIc)

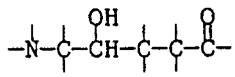 (IId)

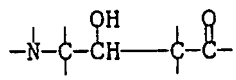 (IIe)

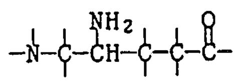 (IIf)

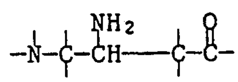 (IIg)

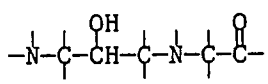 (IIh)

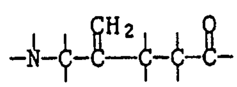 (IIi)

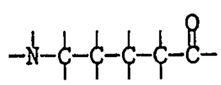 (IIj)

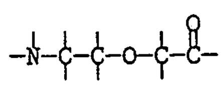 (IIk)

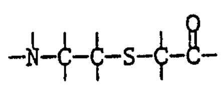 (IIl)

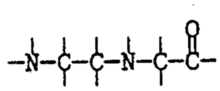 (IIm)

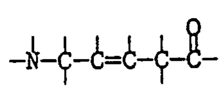 (IIn)

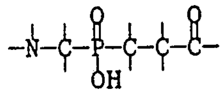 (IIo)

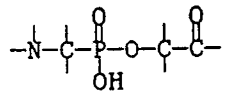 (IIp)

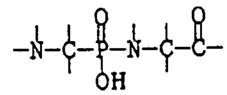 (IIq)

oder 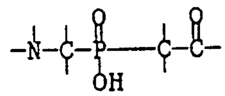 (IIr)

steht, worin freie Valenzen, die nicht an AAN bzw. AAC gebunden sind, unabhängig voneinander durch Wasserstoff, unsubstituiertes oder substituiertes Niederalkyl, Niederalkenyl, Niederalkinyl, Mono-, Bi- oder Tricycloalkyl, Cycloalkylniederalkyl, unsubstituiertes oder substituiertes Aryl, Arylniederalkyl, Arylniederalkenyl, unsubstituiertes oder substituiertes Heteroaryl, Heteroarylniederalkyl, unsubstituiertes oder substituiertes Hydroxy oder unsubstituiertes oder substituiertes Amino abgesättigt und/oder freie Valenzen an benachbarten N- und C-Atomen durch Niederalkylen überbrückt sind.

6. Verwendung gemäss Anspruch 5, dadurch gekennzeichnet, dass in einer der Formeln IIa bis IIr die freien Valenzen, die nicht an AAN bzw. AAC gebunden sind, in einer Weise abgesättigt und/oder überbrückt sind, dass die resultierenden Isostere den Dipeptidresten Leucin-Valin, Phenylalanin-Valin, Cyclohexylalanin-Valin, Leucin-Alanin, Cyclohexylalanin-Alanin, Leucin-Glycin, Cyclohexylalanin-Glycin, Tyrosin-Prolin, Phenylalanin-Prolin oder Cyclohexylalanin-Prolin entsprechen.

7. Verwendung gemäss Anspruch 5, dadurch gekennzeichnet, dass in einer der Formeln IIa bis IIr die freien Valenzen, die nicht an AAN bzw. AAC gebunden sind, in einer Weise mit Wasserstoff, Niederalkyl und/oder Cyclohexylmethyl abgesättigt sind, dass die resultierenden Isostere den Dipeptidfragmenten Leucin-Valin oder Cyclohexylalanin-Valin entsprechen.

8. Verwendung gemäss Anspruch 4, dadurch gekennzeichnet, dass in einer Verbindung der Formel I das Radikal AAN zwei oder mehr α-Aminosäurereste aufweist oder das Radikal AAN aus nur einem Aminosäurerest besteht und gleichzeitig der Rest $R^2$ ein Analogon zu Phenylalanyl ist.

9. Verwendung gemäss Anspruch 4, dadurch gekennzeichnet, dass in einer Verbindung der Formel I das Radikal AAC zwei α-Aminosäurereste aufweist oder das Radikal AAC aus nur einem Aminosäurerest besteht und gleichzeitig der Rest $R^1$ ein Analogon zum über Stickstoff gebundenen Rest der Aminosäure Tyrosin ist.

10. Verwendung gemäss Anspruch 5, dadurch gekennzeichnet, dass in einer Verbindung der Formel I Q einen Rest der Teilformen

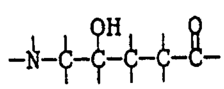 (IId)

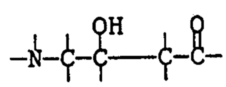 (IIe)

oder 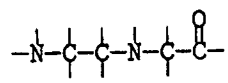 (IIm)

darstellt, worin freie Valenzen, die nicht an AAN bzw. AAC begunden sind, unabhängig voneinander durch Wasserstoff, Niederalkyl, Arylniederalkyl oder Cycloalkylniederalkyl abgesättigt und/oder freie Valenzen an benachbarten N- und C-Atomen durch Niederalkylen überbrückt sind.

11. Verwendung gemäss Anspruch 10, dadurch gekennzeichnet, dass in einer der Formeln IId, IIe oder IIm die freien Valenzen, die nicht an AAN bzw. AAC gebunden sind, in einer Weise abgesättigt und/oder überbrückt sind, dass die resultierenden Isostere den Dipeptidresten Leucin-Valin, Phenylalanin-Valin,

Cyclohexylalanin-Valin, Leucin-Glycin, Cyclohexylalanin-Glycin, Tyrosin-Prolin, Phenylalanin-Prolin oder Cyclohexylalanin-Prolin entsprechen.

12. Verwendung von Verbindungen vom Typus der Renininhibitoren oder verwandter Aspartatproteinaseinhibitoren als gag-Protease-Inhibitoren.